# EUROPEAN PATENT APPLICATION

(11) **EP 4 631 972 A1**
(43) Date of publication of application: **15.10.2025**
(21) Application number: 23890299.3
(22) Date of filing: 10.08.2023
(51) Int. Cl.: C07K 14/55, C07K 17/08, A61K 38/20

(54) **SITE-SPECIFIC COUPLED PEGYLATED INTERLEUKIN-2 MUTANT WITH RECEPTOR AFFINITY PREFERENCE AND USE THEREOF**

(30) Priority: 18.11.2022 CN 202211450658
(71) Applicant: Nanjing Novoacine Biotechnology Co., Ltd., Nanjing, Jiangsu 210032 (CN)
(72) Inventor: ZHONG, Kai, Nanjing, Jiangsu 210032 (CN); WU, Wei, Nanjing, Jiangsu 210032 (CN); QI, Xin, Nanjing, Jiangsu 210032 (CN); XIONG, Xinhui, Nanjing, Jiangsu 210032 (CN); ZHANG, Tao, Nanjing, Jiangsu 210032 (CN); WANG, Qing, Nanjing, Jiangsu 210032 (CN); WANG, Kaiqi, Nanjing, Jiangsu 210032 (CN); YE, Yi, Nanjing, Jiangsu 210032 (CN)
(74) Representative: Bianchetti & Minoja with Trevisan & Cuonzo IPS SRL
(86) International application number: PCT/CN2023/112276
(87) International publication number: WO 2024/103860

(57) **Abstract**

Provided is a site-specific PEG-modified interleukin 2 (IL-2) mutant. Compared with a parent IL-2, the mutant has an improved preference for a Treg cell and a prolonged half-life period. Also provided are a composition comprising the IL-2 mutant, a nucleic acid encoding the IL-2 mutant, a vector comprising the nucleic acid, and a host cell. Further provided are the IL-2 mutant and therapeutic use of a pharmaceutical composition comprising same.

## Description

### FIELD OF THE INVENTION

The present invention relates to a PEGylated interleukin 2 (IL-2) mutant and uses thereof. In particular, the present invention relates to PEGylated IL-2 mutants having improved preference for Treg cells and prolonged half-life compared with the parent IL-2. The present invention also provides compositions comprising the IL-2 mutant, as well as nucleic acids encoding the IL-2 mutants, vectors comprising the nucleic acids, and host cells. The present invention further provides therapeutic uses of the IL-2 mutants and pharmaceutical compositions comprising the same.

### BACKGROUND OF THE INVENTION

Regulatory T cells (Tregs) are a type of CD4+CD25+T cells discovered in the 1970s. They are negative immune regulatory cells whose main function is to regulate immune responses in multiple aspects and maintain immune homeostasis. Treg cells are the core of immune system homeostasis and play a major role in maintaining tolerance to self-antigens and regulating immune responses to foreign antigens. Defects in the number or function of Treg cells have been found in a variety of autoimmune diseases, including rheumatoid arthritis, type 1 diabetes, nephritis, multiple sclerosis, systemic lupus erythematosus, etc. Therefore, developing methods to restore the number or function of Tregs for the treatment of various autoimmune diseases has become a hot topic in recent years.

Interleukin-2 (IL-2) is a multifunctional cytokine expressed on a variety of different immune cell types. It is mainly synthesized by activated T cells, especially helper T cells, and plays biological functions including regulating the body's immune response and enhancing the body's immune monitoring. In eukaryotic cells, human IL-2 (uniprot: P60568) is synthesized as a 153 amino acid precursor polypeptide, which, after removal of the N-terminal 20 amino acids, produces mature secretory IL-2 (comprising 133 amino acids).

In most cases, IL-2 exerts its effects through IL-2 receptor (IL-2R). IL-2R is composed of three subunits: α (CD25), β (CD122), and γ (CD132). Different chains and their combinations form receptor forms with different affinities for IL-2: (1) low-affinity receptor (IL-2Rα; CD25); (2) medium-affinity receptor (IL-2Rβγ); and (3) high-affinity receptor (IL-2Rαβγ). Cytotoxic CD8 ⁺ T cells and natural killer (NK) cells mainly express medium-affinity receptor (IL-2Rβγ), while Treg cells mainly express high-affinity receptor for IL-2 (IL-2Rαβγ) on their cell surface due to the constitutive high expression of CD25.

IL-2 has a dual function in immune response by binding to different IL-2 receptors, that is, it not only mediates the proliferation and activity of effector cells, but is also involved in maintaining peripheral immune tolerance. Specifically, as an immune system stimulator, IL-2 activates the proliferation and differentiation of T cells by binding to the β subunit and the γ subunit, induces the generation of cytotoxic T lymphocytes (CTLs), and stimulates the production, proliferation and activation of natural killer (NK) cells, thereby having anti-tumor activity; on the other hand, it is known that the imbalance between Treg cells and effector T cells (Teff) is the intrinsic cause of various autoimmune diseases, and the IL-2Rα subunit on Treg cells greatly enhances the binding ability of IL-2 to the IL-2 receptor β and γ subunits. Therefore, IL-2 plays a positive role in the treatment of autoimmune diseases.

In order to improve the efficacy of IL-2 in autoimmune diseases, the existing ideas for engineering IL-2 in the prior art include: 1) A method of extending the half-life of natural IL-2 by using PEG, Fc, etc. (for example, CN201980034052). However, due to the bidirectional regulation mechanism of natural IL2, PEGylated natural IL2 cannot completely eliminate the expansion effect on Teff cells and NK cells, thus affecting its therapeutic effect; 2) A method of preparing IL2 mutants to change the preference of IL-2 for receptors. Since IL-2 itself is less stable, its mutants will usually be more unstable. Therefore, the conventional method of random mutation to find molecules with suitable properties is not applicable to IL-2 protein. Even if an IL-2 mutant (such as the mutant disclosed in CN100366742C) is obtained, the developability of the IL-2 is not high due to the short half-life thereof; and 3) A method of preparing IL2 mutants and fusing them with Fc or PEG to increase the half-life thereof, e.g., CN201880081506 discloses a scheme for fusing IL2 mutants with Fc, and CN201580053284 discloses a scheme for fusing IL-2 mutants comprising D109C substitution with PEG. These schemes have been solved the issues to some extent. However, the IL-2 mutants with multiple site mutation combinations disclosed in CN201880081506 have immunogenicity and half-life risks due to the large differences from the natural protein sequence. The PEGylated IL2 mutants comprising N88R+D109C disclosed in CN201580053284 only prolong the half-life compared with the unmodified IL2 (N88R) mutant, while not significantly improve the preference, and the molecules still have significant activation effects on NK cells.

Therefore, there is still a need in the art to develop new IL-2 mutant molecules with better preference for Treg cells and prolonged half-life, especially IL-2 mutant molecules that exhibit favorable properties for production and purification and exhibit improved pharmacodynamic.

The present invention meets these needs to some extent by providing novel PEG site-specifically conjugated IL-2 mutant molecules with improved IL-2 receptor selectivity/preference, prolonged half-life and/or improved developability relative to the parent IL-2.

### SUMMARY OF THE INVENTION

The present application obtains polyethylene glycol site-specifically conjugated interleukin-2 mutants (referred to as PEGylated IL-2 mutants or IL-2 mutants, and in a specific IL-2 mutant molecule, it is sometimes described as a "PEG-IL-2" molecule, which is usually followed by a specific amino acid mutation found in the IL-2 molecule) with improved preference, high Treg activation, and good *in vivo* efficacy based on mutation modification of different sites of the parent IL-2 and subsequent site-specific PEGylation modification.

Therefore, the first aspect of the present application provides a new PEGylated IL-2 mutant, which has one or more of the following advantages:
(i) enhanced binding preference to the receptor IL-2Rαβγ ;
(ii) reduced or eliminated binding to IL-2Rβγ;
(iii) effectively activating STAT5 phosphorylation in Treg cells *in vitro,* but having no activating effect on STAT5 phosphorylation in Teff cells and NK cells;
(iv) activating Treg cells to a higher degree *in vivo,* but having no activating effect on Teff cells and NK cells;
(v) prolonged half-life, thus reducing dosing frequency;
(vi) improved safety of IL-2 mutants due to the optimization of the PEG modification sites;
(vii) having a very small number of mutations to the IL-2 protein, thereby reducing the risk of immunogenicity of the mutant proteins in the body;
(viii) greatly reduced or minimized toxic side effects of the IL-2 mutants since the side effects of IL-2 are mainly caused by the activation of NK cells, while the IL-2 mutants of the present application substantially do not cause NK cell activation due to the reduced or eliminated affinity for IL-2Rβγ.

In some embodiments, the present application provides an IL-2 mutant, which comprises a specific amino acid residue modification relative to the parent IL-2 with natural or non-natural amino acid, and preferably the modification is an amino acid substitution.

In some embodiments, the IL-2 mutants provided herein comprise an amino acid substitution for site-specific conjugation with PEG molecule. Therefore, the IL-2 mutants conjugated with PEG disclosed in the present application are also referred to as PEGylated mutants.

In some embodiments, the PEGylated IL-2 mutants provided herein comprise one or more amino acid mutations at amino acid positions 33, 75, 77, 84, 88, 91 and 109 of the IL-2 amino acid sequence, and the amino acid positions are numbered according to the parent sequence as set forth in SEQ ID NO: 1, wherein the PEG molecule is conjugated to one of amino acids at positions 33, 75, 77, 84, 91, and 109.

In a specific embodiment, the PEG molecule comprises a chemically reactive group for conjugation to a protein, such as a maleimide group, an o-dithiopyridyl group, an azide group, a p-toluenesulfonate group or a methanesulfonate group, a hydroxylamine group (oxyamine). In a specific embodiment, the PEG molecule is conjugated via a cysteine residue, an unnatural amino acid, or any other method disclosed in the prior art. In yet another embodiment, the PEG molecule comprises a chemically reactive group that is conjugated to an amino acid residue comprising an amino group, an alkynyl group, or a free thiol group. In a specific embodiment, the chemically reactive group is a maleimide or iodoacetamide group, which is conjugated to the free thiol group of a cysteine residue. In a specific embodiment, the chemically reactive group is a hydroxylamine group, which is conjugated to a p-acetylphenylalanine residue. In a specific embodiment, the chemically reactive group is an azide group, which is conjugated to N-E-propargyloxycarbonyl-L-lysine.

In a specific embodiment, the PEG molecule can be a linear molecule or a branched molecule.

In a specific embodiment, the PEG molecule has an average molecular weight of 5-100K Da, preferably 20K-100K, more preferably 40k-80k.

In a specific embodiment, the PEGylated IL-2 mutants provided herein comprise one or more amino acid substitutions selected from: N33C, S75C, N77C, D84C, N88C, N88R, D109C and V91C, and the amino acid positions are numbered according to the parent sequence as set forth in SEQ ID NO: 1.

In a specific embodiment, the PEGylated IL-2 mutants provided herein comprise one or more amino acid substitutions selected from: N33Prk, N77Prk or N88R, and the amino acid positions are numbered according to the parent sequence as set forth in SEQ ID NO: 1.

In a specific embodiment, the PEGylated IL-2 mutants provided herein comprise one or more amino acid substitutions selected from: N33pAcF, N77pAcF or N88R, and the amino acid positions are numbered according to the parent sequence as set forth in SEQ ID NO: 1.

In a specific embodiment, in the PEGylated IL-2 mutants provided by the present application, the PEG molecule is conjugated to a cysteine selected from positions 33, 75, 77, 84, 91, and 109 of the IL-2 molecule. Preferably, the PEG molecule is conjugated to one of these cysteines. More preferably, the PEG molecule is conjugated to the sulfhydryl group of the cysteine at the corresponding position through the maleimide group it contains. Most preferably, the PEG molecule is conjugated to the cysteine at position 77 of the IL-2 molecule. In a specific embodiment, each IL-2 molecule is conjugated to one PEG molecule. In a specific embodiment, in the PEGylated IL-2 mutants provided by the present application, the PEG molecule is conjugated to a non-natural amino acid (*e.g.,* p-acetylphenylalanine) selected from positions 33, 75, 77, 84, 91 and 109 of the IL-2 molecule. Preferably, the PEG molecule is conjugated to one of the non-natural amino acids (*e.g.,* p-acetylphenylalanine). More preferably, the PEG molecule is conjugated to the non-natural amino acid (*e.g.,* p-acetylphenylalanine) at the corresponding position through the hydroxylamine group it comprises. Most preferably, the PEG molecule is conjugated to the non-natural amino acid (*e.g.,* p-acetylphenylalanine) at position 77 of the IL-2 molecule. In a specific embodiment, each IL-2 molecule is conjugated to one PEG molecule. In a specific embodiment, in the PEGylated IL-2 mutants provided herein, the PEG molecule is conjugated to a non-natural amino acid (*e.g.,* N-E-propargyloxycarbonyl-L-lysine) selected from positions 33, 75, 77, 84, 91 and 109 of the IL-2 molecule. Preferably, the PEG molecule is conjugated to one of the non-natural amino acids (*e.g.,* N-E-propargyloxycarbonyl-L-lysine). More preferably, the PEG molecule is conjugated to the non-natural amino acid (*e.g.,* N-E-propargyloxycarbonyl-L-lysine) at the corresponding position through the azide group it comprises. Most preferably, the PEG molecule is conjugated to the non-natural amino acid (*e.g.,* N-E-propargyloxycarbonyl-L-lysine) at position 77 of the IL-2 molecule. In a specific embodiment, each IL-2 molecule is conjugated to one PEG molecule.

In a specific embodiment, the PEGylated IL-2 mutants provided herein comprise the mutation N88R, and the PEG molecule is conjugated to a cysteine residue or a non-natural amino acid (*e.g.,* p-acetylphenylalanine, N-E-propargyloxycarbonyl-L-lysine) selected from positions 33, 75, 77 and 109 of the IL-2 molecule.

In a specific embodiment, the PEGylated IL-2 mutants provided herein comprise the mutation N88R, and the PEG molecule is conjugated to a cysteine residue or a non-natural amino acid (*e.g.,* p-acetylphenylalanine, N-E-propargyloxycarbonyl-L-lysine) at position 77 of the IL-2 molecule. In a preferred embodiment, the PEG molecule is a linear molecule. In a preferred embodiment, the PEG molecule has an average molecular weight of 5-100K Da, preferably an average molecular weight of 20K-100K, and more preferably an average molecular weight of 40k-80k.

In one embodiment, the PEGylated IL-2 mutants provided herein have a prolonged half-life of at least 3 hours, at least 5 hours, at least 10 hours, at least 15 hours, at least 20 hours, at least 1 day, at least 2 days, at least 3 days, at least 4 days, at least 5 days, at least 6 days or at least 9 days. This prolonged half-life allows for a reduced dosing frequency of the IL-2 mutant of the present application.

In one embodiment, the PEGylated IL-2 mutants provided herein have a reduced binding affinity to the IL-2Rβγ receptor relative to the parent IL-2, for example, the STAT5 reporter gene signal value at high concentration is reduced to 5%-50%, preferably, 15%-30%.

In one embodiment, the PEGylated IL-2 mutants provided herein increase IL-2-mediated CD25⁺ cell activation and/or proliferation relative to the parent IL-2. In one embodiment, the CD25⁺ cells are Treg cells. In one embodiment, the ability of IL-2 mutants to activate Treg cells is identified by detecting changes in the number of Treg cells expanded by IL-2 mutants in a wild-type mouse pharmacodynamic (PD) test. In one embodiment, the IL-2 mutants of the present invention increase the number of the expanded Treg cells by at least 1-fold, such as 2-fold, 3-fold, 4-fold, 5-fold, 10-fold, or 15-fold relative to the parent IL-2 protein as measured in a wild-type mouse pharmacodynamic (PD) test. In another embodiment, the ability and preference of IL-2 mutants to activate Treg cells are identified by detecting changes in the expansion of Treg, NK, and CD8+T cells by IL2 mutants in cynomolgus monkey pharmacodynamic and pharmacokinetic tests. The IL-2 mutants of the present invention increase the ratio of the expanded Treg cells in cynomolgus monkeys to CD3+T cells by at least 1-fold, such as 2-fold, 10-fold, 20-fold, 30-fold, 50-fold, 75-fold or 90-fold. The ratio of the expanded NK cells in cynomolgus monkey to PBMCs does not exceed 2-fold, and the ratio of the expandedCD8+T cells in cynomolgus monkey to CD3+T cells does not exceed 2-fold.

In one embodiment, the IL-2 mutants of the present invention eliminate or reduce the activation of CD8⁺ T cells and NK cells by IL-2 relative to the parent IL-2.

In another embodiment, the IL-2 mutants of the present invention may also be conjugated with other non-immunogenic polymers. Such polymers are recombinant non-immunogenic amino acid polymers such as XTEN polymers, PAS polymers and elastin-like polypeptides (ELPs), among others. XTEN comprises chains with A, E, G, P, S and T amino acids expressed by using *E. coli* (Schellenberger, V., et al., 2009, Nat Biotechnol. 27: 1186-90). PAS polymers are unstructured peptide polymers composed of repeating sequences of P, A and S amino acid residues (Schlapschy, M., et al., 2007, Protein Eng Des Sel. 20:273-84). Elastin-like polypeptides (ELPs) are random sequences containing V-P-G-x-G repeats, found primarily in elastin, where x represents any protein other than proline (Doreen M. Floss et al., 2010, Trends Biotechnol. 28:37-45).

In one embodiment, non-immunogenic polymers may serve as alternatives to PEG molecules for conjugation with the aforementioned IL-2 mutants.

In a second aspect, the present application provides pharmaceutical compositions and combination products comprising the IL-2 mutant.

In one embodiment, the pharmaceutical composition further comprises a pharmaceutically acceptable carrier.

In a third aspect, the present application provides nucleic acids encoding the IL-2 mutants of the present application, and vectors and hosts cell comprising the nucleic acid.

In a fourth aspect, the present application provides a method for preparing the PEGylated IL-2 mutants, including the steps of culturing the host cell of the third aspect under conditions suitable for expressing the IL-2 mutants with a specific amino acid mutation, recovering the expressed mutants, and performing a site-specific PEGylation on the IL-2 mutants.

In a fifth aspect, the present application also provides a method for treating a disease or reducing organ transplant rejection by using the IL-2 mutants and pharmaceutical compositions of the present invention.

In a sixth aspect, the present application provides the use of the IL-2 mutants and pharmaceutical compositions of the present invention in the preparation of a medicament for treating a disease or reducing organ transplant rejection.

In one embodiment, the present application provides the IL-2 mutants and pharmaceutical compositions of the present invention for use in treating a disease.

In one embodiment of the fifth aspect and the sixth aspect, the disease is an inflammatory disease (e.g., autoimmune disease, ankylosing spondylitis, amyotrophic lateral sclerosis, hepatitis C associated vasculitis, sclerosing cholangitis, inflammatory myopathy, relapsing polychondritis, Behcet's disease), coronary artery disease, macrophage activation syndrome, acute lung injury, hypertension, Takayasu disease, Duchenne muscular dystrophy, transient ischemic stroke, ischemic heart disease, Wiskott-Aldrich syndrome, bone marrow transplantation, transplantation indications *(e.g.,* corneal transplantation, islet transplantation, skin transplantation). In a specific embodiment, the autoimmune disease is rheumatoid arthritis, autoimmune encephalitis, type I diabetes, nephritis, multiple sclerosis, systemic lupus erythematosus, cutaneous lupus erythematosus, lupus nephritis, Sjögren's syndrome, psoriasis, plaque psoriasis, alopecia areata, dermatomyositis, scleroderma, myasthenia gravis, demyelinating disease, inflammatory bowel disease, ulcerative colitis, Crohn's disease, autoimmune glomerulonephritis, pulmonary renal hemorrhagic syndrome, graft-versus-host disease, organ transplant rejection, autoimmune hepatitis, autoimmune vasculitis, atopic dermatitis, autoimmune hemolytic anemia, idiopathic thrombocytopenic purpura or idiopathic leukopenia. In a specific embodiment, the autoimmune disease is rheumatoid arthritis, type I diabetes, nephritis, multiple sclerosis, systemic lupus erythematosus, cutaneous lupus erythematosus, lupus nephritis, dermatomyositis, scleroderma, myasthenia gravis, inflammatory bowel disease, ulcerative colitis, Crohn's disease, atopic dermatitis.

In a seventh aspect, the present application provides a method for improving IL-2 receptor activation preference and significantly extending the half-life of the IL-2 mutant, including conjugating PEG to the cysteine residue at position 77.

In an eighth aspect, the present application provides a method for selectively stimulating regulatory T cells, including contacting a T cell population or peripheral blood with the above PEGylated IL-2 mutant, thereby selectively stimulating STATS phosphorylation of regulatory T cells compared to non-regulatory T cells or NK cells.

In one embodiment, the present application provides a method for selectively stimulating regulatory T cells in a subject, including administering the above PEGylated IL-2 mutant to the subject *(e.g.,* a rodent or a non-human primate), to selectively stimulate the proliferation of regulatory T cells compared to non-regulatory T cells or NK cells. In one embodiment, the above method increases the ratio of regulatory T cells to non-regulatory T cells, or increases the ratio of regulatory T cells to NK cells, compared to pre-administration.

The present invention is further illustrated in the following figures and specific embodiments. However, these drawings and specific embodiments should not be considered to limit the scope of the present invention, and modifications that are easily conceivable to those skilled in the art will be included within the spirit of the present invention and the scope of protection of the appended claims.

### DESCRIPTION OF THE DRAWINGS

Fig. 1 shows the signal values of the IL-2βγ dual receptor activated by each PEG-IL-2 mutant at a concentration of 65.2 nM.
Fig. 2 shows the activity of each PEG-IL-2 mutant in activating IL-2βγ dual receptor at different concentrations.
Fig. 3 shows the activity of each PEG-IL-2 mutant in activating IL-2αβγ triple receptor at different concentrations.
Fig. 4 shows the activity of each PEG-IL-2 mutant in activating IL-2βγ dual receptor at different concentrations.
Fig. 5 shows the normalized signal values of βγ dual receptor activated by each PEG-IL-2 mutant at a concentration of 250 nM.
Fig. 6 shows the activity of each PEG-IL-2 mutant in activating IL-2αβγ triple receptor at different concentrations.
Fig. 7 shows the effects of 80K-1PEG-IL2-N88R-N77C and 80K-1PEG-IL2-N88R-D109C on the body weight of wild-type mice after administration.
Fig. 8 shows the SDS-PAGE profile of IL-2-N88R-N77C modified with PEGs of different molecular weights, left: Coomassie brilliant blue staining, right: iodine staining, Lane 1: Marker, Lane 2: 80K-1PEG-IL2-N88R-N77C, Lane3: 60K-1PEG-IL2-N88R-N77C, Lane4: 40K-1PEG-IL2-N88R-N77C, Lane5: 20K-1PEG-IL2-N88R-N77C.
Fig. 9 shows the activation of human Treg cells by IL2-N88R-N77C mutants modified with PEGs of different molecular weights.
Fig. 10 shows the activation of human NK cells by IL2-N88R-N77C mutants modified with PEGs of different molecular weights.
Fig. 11 shows the activation of human CD8+ T cells by IL2-N88R-N77C mutants modified with PEGs of different molecular weights.
Fig. 12 shows the increases in peripheral Treg cells in mice after a single administration of IL2-N88R-N77C mutants modified with PEGs of different molecular weights.
Fig. 13 shows the effects of IL2-N88R-N77C mutants modified with PEGs of different molecular weights on peripheral CD8+ T cells in mice after a single administration.
Fig. 14 shows the effects of IL2-N88R-N77C mutants modified with PEGs of different molecular weights on peripheral NK cells in mice after a single administration.
Fig. 15 shows the PK concentration-time curves in mice after a single administration of IL2-N88R-N77C mutants modified with PEGs of different molecular weights.
Fig. 16 shows the effects of IL2-N88R-N77C mutants modified with PEGs of different molecular weights on ear thickness in DTH mouse model.
Fig. 17 shows the concentration-time curves in cynomolgus monkeys after administration of IL2-N88R-N77C mutants modified with PEGs of different molecular weights.
Fig. 18 shows the effects of IL2-N88R-N77C mutants modified with PEGs of different molecular weights on peripheral NK cells in cynomolgus monkeys after administration.
Fig. 19 shows the increases in peripheral Treg cells in cynomolgus monkeys after administration of IL2-N88R-N77C mutants modified with PEGs of different molecular weights.
Fig. 20 shows the effects of IL2-N88R-N77C mutants modified with PEGs of different molecular weights on peripheral CD8+T cells in cynomolgus monkeys after administration.
Fig. 21 shows the increases in the expression intensity of CD25 protein on peripheral Treg cells in cynomolgus monkeys after administration of IL2-N88R-N77C mutants modified with PEGs of different molecular weights.
Fig. 22 shows the increases in the expression intensity of Foxp3 protein in peripheral Treg cells in cynomolgus monkeys after administration of IL2-N88R-N77C mutants modified with PEGs of different molecular weights.
Fig. 23 A-E show the effects of IL2-N88R-N77C mutants modified with PEGs of different molecular weights on some hematological and blood biochemical indicators (Na+, Cl-, EOS, ALB, CRP) in cynomolgus monkeys after administration.
Fig. 24A-C show the binding preference of each of the PEG-IL-2 mutants with phenylalanine analog mutations to the IL-2 receptor, Figure 24A shows the activity of activating the IL-2βγ dual receptor at different concentrations; Figure 24B shows the normalized signal value of each of the PEG-IL-2 mutants activating the IL-2βγ dual receptor at a concentration of 250 nM; Figure 24C shows the activity of each of the PEG-IL-2 mutants activating the IL-2αβγ triple receptor at different concentrations.
Fig. 25A-C show the binding preference of each of the PEG-IL-2 mutants with lysine analog mutations to the IL-2 receptor, Figure 25A shows the activity of activating the IL-2βγ dual receptor at different concentrations; Figure 25B shows the normalized signal value of each of the PEG-IL-2 mutants activating the IL-2βγ dual receptor at a concentration of 250 nM; Figure 25C shows the activity of each of the PEG-IL-2 mutants activating the IL-2αβγ triple receptor at different concentrations.

### DETAILED DESCRIPTION OF THE INVENTION

Unless otherwise defined, all technical and scientific terms used herein have the same meanings as commonly understood by one of ordinary skill in the art. For purposes of the present invention, the following terms are defined below.

The term "about" when used in connection with a numerical value is meant to encompass numerical values within the range between the lower limit of 5% less than the specified numerical value and the upper limit of 5% greater than the specified numerical value.

The term "and/or" should be understood to mean any one of the optional items or a combination of any two or more of the optional items.

As used herein, the term "comprising" or "including" means to include the stated elements, integers or steps, but does not exclude any other elements, integers or steps. When the term "comprising" or "including" is used herein, unless otherwise specified, it also encompasses the situation consisting of the stated elements, integers or steps. For example, when reference is made to an IL-2 mutant that "comprising" or "includes" a certain mutation or combination of mutations, it is also intended to cover IL-2 mutants having only said mutation or combination of mutations.

The term parent "interleukin-2" is used interchangeably with parent "IL-2" and parent "IL2", and refers to a parent IL-2 protein used as a template for introducing a mutation or combination of mutations of the present invention, preferably refers to a naturally occurring IL-2 protein, such as a natural IL-2 protein from humans, mice, rats, or non-human primates, preferably human IL-2, including unprocessed *(e.g.,* with signal peptide) and processed *(e.g.,* signal peptide removed) forms, preferably a mature IL-2 protein, such as a mature human IL-2 protein (uniprot: P60568). Furthermore, the parent "interleukin-2" also includes naturally occurring IL-2 allelic variants and splice variants, isoforms, homologs, and species homologs. In some embodiments, the parent IL-2 may contain amino acid mutations that do not affect its binding to the IL-2 receptor compared to the natural IL-2 protein. For example, a human IL-2 protein in which a substitution has been introduced at position 125 belongs to the parent IL-2 of the present invention, such as C125S and C125A. Said substitution eliminates the unpaired cysteine residue at position 125 in IL-2 and promotes the stability of IL-2 and avoids the formation of IL-2 dimers; for example, a human IL-2 protein lacking alanine at position 1; and a combination of the above mutations, such as IL-2 lacking alanine at position 1 and simultaneously having substitution C125S.

Depending on the system used to express a protein having IL-2 activity, the IL-2 protein may be unglycosylated or glycosylated, and either may be used. That is, the IL-2 protein can be unglycosylated, or the IL-2 protein can be glycosylated, and in one or more preferred embodiments, the IL-2 protein is unglycosylated. The IL-2 protein may also advantageously be modified to include and/or substitute one or more amino acid residues, such as, for example, lysine, cysteine and/or arginine, to provide a site for attachment to a polymer via an atom within the side chain of the modified amino acid. Examples of substitutions of IL-2 proteins are described in US Pat. No. 5,206,344. Additionally, the IL-2 protein can be modified to include non-naturally occurring amino acid residues. Techniques for adding amino acid residues and non-naturally occurring amino acid residues are well known to those of ordinary skill in the art.

The amino acid mutation may be an amino acid substitution, deletion, insertion or addition. In some embodiments, the amino acid mutation is a substitution of one or more amino acids, such as a single amino acid substitution or a combination of multiple amino acid substitutions. Amino acid deletions and insertions include those occurring at the amino-terminus and/or carboxyl-terminus of the polypeptide sequence, as well as those occurring within the internal region of the polypeptide sequence. Amino acid substitutions of the present invention optionally include conservative substitutions of amino acids.

Amino acid substitutions referred to herein are described in the following manner: single amino acid substitution: (original amino acid residue/position/substituted amino acid residue). For example, the substitution of asparagine at position 88 to arginine can be represented as N88R. Accordingly, individual amino acid substitutions can be linked by the symbol "-" to indicate combined mutations at multiple specified positions. For example, a combined mutation of positions N88R and D109C is expressed as: N88R-D109C.

When referring to amino acid positions of the IL-2 protein or IL2 sequence, reference is made to the amino acid sequence of parent mature human IL-2 protein as set forth in SEQ ID NO: 1. The corresponding amino acid positions in other IL-2 proteins or polypeptides (including full-length sequences or truncated fragments) can be identified by aligning the amino acid sequence with SEQ ID NO: 1. Therefore, in the present invention, unless otherwise specified, the amino acid positions of IL-2 proteins or polypeptides are numbered according to SEQ ID NO: 1. For example, when referring to "N88", it refers to the asparagine residue N at position 88 of SEQ ID NO: 1, or the amino acid residue at the corresponding position in other IL-2 polypeptide sequences after alignment.

The "percent sequence identity" can be determined by comparing two optimally aligned sequences over the comparison window. Preferably, the sequence identity can be determined over the entire length of the reference sequence (*e.g.,* SEQ ID NO:1). Methods of alignment of sequences for comparison are well known in the art. Suitable algorithms for determining percent sequence identity include, for example, BLAST and BLAST 2.0 algorithms (see Altschul et al., Nuc. Acids Res. 25: 3389-402,1977 and Altschul et al. J. Mol. Biol. 215: 403-10, 1990). Software for performing BLAST analyses is publicly available through the National Center for Biotechnology Information. For the purposes of this application, percent identity is determined using the Basic Local Alignment Search Tool available from https://blast.ncbi.nlm.nih.gov/Blast.cgi using default parameters.

"Affinity" or "binding affinity" refers to the intrinsic binding capacity that reflects the interaction between members of a binding pair. The affinity of a molecule X for its binding partner Y can be expressed by the equilibrium dissociation constant (K_{D}), which is the ratio of the dissociation rate constant and the association rate constant (k_{dis} and kₒₙ, respectively). Binding affinity can be measured by common methods known in the art.

Polyethylene glycol (PEG) is a water-soluble polymer of ethylene glycol, and it has the advantages of low toxicity, good amphiphilicity, low immunogenicity and biocompatibility. PEG is available as linear or branched molecules of varying sizes and can also be modified to have a chemically reactive group for conjugation to a protein. Various forms of PEG molecules have been developed as commercial products. Modification of proteins with PEG includes random modification (for example, a mixture of polyethylene glycol random modifications is often obtained by using ε-NH₂ or α-NH₂ of lysine as the modification site, since proteins usually contain multiple lysines) and site-specific modification. Site-specific modification usually involves modifying cysteine in a protein. Most cysteine residues in the protein form disulfide bonds to stabilize protein conformation, but a small amount of free (unpaired) cysteine residues are usually buried within the protein interior. In order to conjugate PEG to free cysteine in a protein, it is generally necessary that the protein has a naturally exposed free cysteine residue or has a newly introduced free cysteine residue. When a free cysteine is newly introduced, the protein usually faces the following risks: on the one hand, the newly introduced cysteine can form an incorrect intrachain disulfide bond with other cysteine in the protein, thereby causing protein misfolding; on the other hand, it can form interchain disulfide bond with other molecule, thereby causing protein aggregation. For example, Wang *et al.* have found that mutated cysteine residues in IL-2 lead to the reduced IL-2 activity due to mispaired disulfide bonds (Wang, A., et al., 1984 Science. 224: 1431-3).

"PEGylated IL-2 mutant" refers to an IL-2 protein to which PEG is covalently conjugated, wherein the IL-2 protein can be wild-type IL-2 (*e.g.,* human IL-2 molecule), an IL-2 molecule with a mutation (e.g., human IL-2 with C125S substitution, human IL-2 with N88R substitution). Wild-type IL-2 has a short half-life *in vivo,* and high doses of IL-2 or multiple frequent administrations are usually required to maintain the required amount of IL-2 in the circulation. Therefore, it is necessary to modify wild-type IL-2 to increase its serum half-life. The PEGylated IL-2 mutants of the present application increase the half-life of IL-2, so that the IL-2 protein can be administered at a low dose or less frequently when Treg cells are effectively stimulated, thereby making IL-2 less toxic to the subject, and the subject is more tolerable to IL-2 and has better compliance with IL-2 therapy.

### Polynucleotides, vectors and hosts

The present invention provides nucleic acids encoding any of the above IL-2 mutants or PEGylated conjugates. The polynucleotide sequences encoding the mutant proteins of the present invention can be generated by *de novo* solid phase DNA synthesis or by PCR mutagenesis of an existing sequence encoding the parent IL-2 by using methods well known in the art. In addition, the polynucleotides and nucleic acids of the present invention may contain a segment encoding a secretory signal peptide, which may be operably linked to a segment encoding the mutant protein of the present invention, thereby directing the secretory expression of the mutant protein of the present invention.

The invention also provides vectors comprising the nucleic acid of the invention. In one embodiment, the vector is an expression vector, such as a eukaryotic expression vector and a prokaryotic expression vector. In a preferred embodiment, the expression vector of the present invention is, for example, a pcDNA3.1 expression vector, a pBV220 expression vector, a p1128 expression vector, or a pET30a(+) expression vector.

The present invention also provides prokaryotic and eukaryotic host cells comprising the nucleic acid or the vector. Host cells suitable for replicating and supporting the expression of the mutant IL-2 proteins or PEGylated conjugates are well known in the art. Such cells can be transfected or transduced with a particular expression vector, and large numbers of vector-containing cells can be grown for inoculation of large-scale fermenters, thereby obtaining sufficient quantities of the IL-2 mutants or PEGylated conjugates for clinical use. In one embodiment, the host cell is prokaryotic, such as *E. coli,* see Fischer et al. (1995) Biotechnol. Appl. BioIL-2m. 21(3):295-311]. In another embodiment, the host cell is eukaryotic. In another embodiment, the host cell is selected from yeast cells, mammalian cells (eg, CHO cells or 293 cells). For example, the polypeptides can be produced in bacteria, especially when glycosylation is not desired. Following expression, the polypeptides can be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungal and yeast strains in which glycosylation pathways have been "humanized," resulting in the production of polypeptides with a partially or fully human glycosylation pattern. See Gerngross, Nat Biotech 22, 1409-1414 (2004) and Li et al., Nat Biotech 24, 210-215 (2006).

### Pharmaceutical compositions and pharmaceutical formulations

The present invention also includes compositions (including pharmaceutical compositions or pharmaceutical formulations) comprising the IL-2 mutant or a PEGylated conjugate and compositions comprising the polynucleotide encoding the IL-2 mutant or a PEGylated conjugate thereof. These compositions may also optionally comprise a suitable pharmaceutical adjuvant, such as pharmaceutical carrier, pharmaceutical excipient, including buffer, known in the art.

Pharmaceutical carriers suitable for use in the invention can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Regarding the use of excipients and their uses, see also "Handbook of Pharmaceutical Excipients", Fifth Edition, R.C. Rowe, P.J. Seskey and S. C. Owen, Pharmaceutical Press, London, Chicago. If desired, the composition can also comprise minor amounts of wetting or emulsifying agents, or pH buffering agents. These compositions can take the form of solutions, suspensions, emulsion, tablets, pills, capsules, powders, sustained-release formulations and the like. Oral preparations can include standard carriers such as pharmaceutical grades of mannitol, lactose, starch, magnesium stearate, and saccharin.

The pharmaceutical formulations of the present invention can be prepared by mixing the IL-2 mutant or PEGylated conjugate of the present invention having the desired purity with one or more optional pharmaceutical excipients (Remington's Pharmaceutical Sciences, 16th edition, Osol, A. ed. (1980)), preferably in the form of a lyophilized preparation or an aqueous solution. Exemplary lyophilized antibody formulations are described in US Pat. No. 6,267,958. Aqueous antibody formulations include those described in US Pat. No. 6,171,586 and WO 2006/044908, formulation of the latter includes a histidine-acetate buffer. Additionally, sustained release formulations can be prepared. Suitable examples of sustained release formulations include semipermeable matrices of solid hydrophobic polymers containing the protein, and the matrices are in the form of shaped articles, *e.g.* films, or microcapsules.

In one embodiment, the pharmaceutical compositions of the present invention comprise a buffer at pH 6-8, such as PBS buffer or histidine buffer. In one embodiment, the PBS buffer is, for example, a PBS buffer at about pH 7.4. In one embodiment, the histidine buffer is a histidine buffer at about pH 6.5, for example, comprising 10 mM histidine, 5% sorbitol, and 0.02% polysorbate 80. The pharmaceutical compositions of the present invention preferably remain storage stable in said buffer.

The pharmaceutical compositions or formulations of the present invention may also contain one or more other active ingredients as required for the particular indication being treated, preferably those that do not adversely affect the complementary activities of each other. For example, it may be desirable to also provide other active ingredients for treating autoimmune diseases, suitably combined in amounts effective for the intended use.

### Treating methods and uses

As used herein, the terms "individual" or "subject" are used interchangeably to refer to a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In particular, the subject is a human being.

As used herein, the term "treatment" refers to clinical intervention intended to alter the natural course of a disease in the individual being treated. Desired therapeutic effects include, but are not limited to, preventing disease appearance or recurrence, alleviating symptoms, reducing any direct or indirect pathological consequences of the disease, preventing metastasis, reducing the rate of disease progression, ameliorating or palliating the disease state, and alleviating or improving prognosis.

In one aspect, the present invention provides a method of suppressing the immune system in a subject, the method comprising administering to the subject an effective amount of a pharmaceutical composition comprising a IL-2 mutant or PEGylated conjugate of the present application. The IL-2 mutant of the present invention has high activity and selectivity for Treg cells and has reduced stimulatory effects on cytotoxic CD8 ⁺ T cells and NK cells. Therefore, the IL-2 mutants of the present invention can be used at low doses to suppress the immune system of a subject.

Therefore, in some embodiments, the present invention provides a method for suppressing an immune response in a subject, the method comprising administering to the subject an effective amount of any IL-2 mutant or PEGylated conjugate thereof of the present application. In some embodiments, the IL-2 mutant of the present invention or its PEGylated conjugate is administered to a subject suffering from an autoimmune disease to suppress the immune response thereof.

In another aspect, the present invention relates to a method of treating a disease, such as an autoimmune disease, in a subject, comprising administering to the subject an effective amount of any IL-2 mutant or PEGylated conjugate thereof or a pharmaceutical composition described herein.

The mutant proteins of the invention (and pharmaceutical compositions comprising the same or PEGylated conjugates thereof, and optionally additional therapeutic agents) can be administered by any suitable method, including parenteral, intrapulmonary and intranasal administration, and, if desired for local treatment, intralesional administration. Parenteral infusions include intramuscular, intravenous, intraarterial, intraperitoneal or subcutaneous administration. Administration can be by any suitable route, such as by injections, for example, intravenous or subcutaneous injections, depending in part on whether the administration is short or long term. Various dosing schedules are contemplated herein, including, but not limited to, single dosing or multiple dosing over multiple time points, bolus dosing, and pulse infusion.

For the prevention or treatment of a disease, the appropriate dosage of the mutant proteins of the invention (when used alone or in combination with one or more other therapeutic agents) will depend on the type of disease to be treated, the severity and course of the disease, whether administration is for preventive or therapeutic purposes, previous therapy, the patient's clinical history, and the judgment of the attending physician.

In another aspect, the present invention also provides use of the IL-2 mutant, composition, or PEGylated conjugate of the present invention in the preparation of a medicament for use in the above method (*e.g.,* for treatment). In one embodiment, the present invention also provides the IL-2 mutant, composition, and PEGylated conjugate of the present invention for use in treating a disease.

The following examples are described to aid understanding of the present invention. The examples are not intended to, and should not be interpreted in any way as, limiting the scope of protection of the present invention.

### EXAMPLES

The experimental methods described in the following examples, unless otherwise specified, are all conventional methods in the art using default parameters, steps, etc.; the experimental materials used, unless otherwise specified, are all commercially available products. In the examples where specific techniques or conditions are not described, the procedures follow those disclosed in the literature in the relevant field or are conducted according to the instructions provided in the corresponding product manuals. Reagents or instruments without specified manufacturers are all conventional products that are commercially available through standard channels.

### Example 1: Construction, expression and purification of interleukin-2 mutants

Based on the protein sequence of human mature IL-2 (C125S) (SEQ ID NO:1), cDNAs encoding IL-2 mutants with each type of amino acid mutations listed in Table 1 were designed according to the mutation sites indicated therein. A start codon and an EcoRI restriction site were added to the 5' end of each cDNA, and a stop codon and a BamHI restriction site were added to the 3' end. The cDNAs were prepared by DNA synthesis and subsequently cloned into the temperature-inducible expression vector p1128. The calcium chloride method was used to transform DH5α competent cells with the expression vector p1128 containing the corresponding cDNA sequence, and positive clones were picked from the kanamycin resistance plate and used as expression strains for each mutant protein after sequencing verification.

SEQ ID NO: 1 (IL-2, comprising C125S)
APTSSSTKKTQLQLEHLLLDLQMILNGINNYKNPKLTRMLTFKFYMPKKATELKHLQCLEE
ELKPLEEVLNLAQSKNFHLRPRDLISNINVIVLELKGSETTFMCEYADETATIVEFLNRWITF
SQSIISTLT

**Table 1. List of IL-2 mutants**

| Mutant name | Mutation site | SEQ ID NO: |
|---|---|---|
| IL2-V91C | V91C | SEQ ID NO: 2 |
| IL2-D84C | D84C | SEQ ID NO: 3 |
| IL2-N88C | N88C | SEQ ID NO: 4 |
| IL2-N88R-N33C | N88R, N33C | SEQ ID NO: 5 |
| IL2-N88R-S75C | N88R, S75C | SEQ ID NO: 6 |
| IL2-N88R-N77C | N88R, N77C | SEQ ID NO: 7 |
| IL2-N88R-D109C | N88R, D109C | SEQ ID NO: 8 |

The expression strain of each IL-2 mutant protein was inoculated into TB culture medium (Terrific Broth), cultured in a shaker at 35°C and 250 rpm until the logarithmic growth phase, the temperature was raised to 42°C to induce the expression of each mutant protein for 4 hours, and the bacterial liquid was harvested by centrifugation at 13900 g. The bacteria were washed and resuspended with 50 mM Tris-HCl, 5 mM EDTA, pH 8.0 buffer, disrupted at 700-1000 bar pressure, and the inclusion bodies were harvested by centrifugation at 13900 g.

The IL-2 mutant protein in the form of inclusion body was then renatured and purified. Specifically, the inclusion bodies were washed with a washing buffer containing 2% Triton X-100, and the precipitate was collected after centrifugation. The washed inclusion bodies were dissolved in 7M guanidine hydrochloride solution. After reducing the concentration of guanidine hydrochloride to 2 M, the supernatant was centrifuged and collected. The supernatant was dialyzed overnight against 10 mM NaAc (pH 4.5) containing 5% trehalose, and the buffer was replaced once during the process. After renaturation, the solution was filtered to remove the precipitate, and then purified using CM Sepharose FF ion exchange chromatography and 0-500 mM NaCl solution. The buffers used for CM Sepharose FF ion exchange chromatography were as follows: buffer A: 30 mM PB phosphate buffer, pH 6.5, buffer B: 30 mM PB phosphate buffer, 1 M NaCl, pH 6.5. The chromatography column was first equilibrated with 3 to 5 column volumes of buffer A, and then the renaturation solution was loaded onto the chromatography column. After loading, the chromatography column was rinsed with buffer A, and then gradient eluted with 10 column volumes of 0 to 50% buffer B to collect the purified samples.

Thus, various IL-2 mutants having specific mutation sites as described in Table 1 were obtained. Each IL-2 mutant was named based on its mutation site, as shown in Table 1. For example, the mutant IL2-N88C represents an IL-2 mutant in which a cysteine is replaced for the amino acid asparagine at position 88 of human IL-2 protein sequence, while the mutant IL2-N88R-N33C represents an IL-2 mutant in which an arginine is replaced for the amino acid asparagine at position 88 and a cysteine is replaced for the amino acid asparagine at position 33 of the human IL-2 protein sequence.

### Example 2: Polyethylene glycol modification and purification of IL-2 mutants

Each of IL-2 mutants obtained in Example 1 was modified with polyethylene glycol. The PEG used in this example contains a maleimide group, which can react specifically with the cysteine residue at a specific mutation site of the mutant, thereby allowing the PEG to site-specifically modify the corresponding IL-2 mutant. Specifically, the purified IL-2 mutant proteins were taken and were adjusted to pH 6.5, and the protein concentration to 3 mg/ml. PEG-maleimide (e.g., PEG with Mw80 kDa, Xiamen Sinopeg) was added at a molar ratio of IL-2 mutant: PEG of 1:5, and the reaction was carried out at 2-8°C overnight.

The reaction solution was purified using GE MacroCap SP ion exchange column: the reaction solution was diluted 10 times with ultrapure water, the pH value was adjusted to 5.2, and the sample was loaded onto the chromatography column. The buffers used for the MacroCap SP ion exchange column were as follows: Buffer A: 40 mM sodium acetate, pH 5.2, Buffer B: 40 mM sodium acetate , 1 M NaCl, pH 5.2. The chromatography column was first equilibrated with 3 to 5 column volumes of buffer A, and then the obtained PEGylated IL-2 mutant solution was loaded onto the chromatography column. After loading, the chromatography column was rinsed with buffer A, and then gradient eluted with 10 column volumes of 0 to 50% buffer B to collect and verify the PEG-modified IL-2 mutant protein. The modified mutant proteins were named based on the following convention: PEG MW-nPEG-IL-2-amino acid substitution, where n represents the number of PEGs conjugated to one IL-2 molecule. For example, the IL-2 mutant 80k-1PEG-IL-2-V91C refers to an IL-2 mutant in which one PEG molecule with a molecular weight of 80k is conjugated to the substituted cysteine residue at amino acid position 91 of the IL-2 molecule.

### Example 3. Binding preference of different PEG-modified IL-2 mutants to IL-2 receptor

In this example, the IL-2 mutants with PEG modifications at different sites, obtained from Example 2, were tested for their binding to various IL-2 receptors, and the biological activities of the corresponding mutants were also tested.

Since the binding of IL-2 to the β and γ subunits of its receptor induces STAT5 phosphorylation, which subsequently leads to dimerization and translocation into the nucleus to promote target gene transcription, this example evaluates the ability of the mutant IL-2 polypeptides to induce signaling through the IL-2 receptor by measuring STAT5 phosphorylation.

### 1. Construction of 293EC18/βγ/JAK3/STAT5 and 293EC18/αβγ/JAK3/STAT5 cells

Adherent 293EC18 cells were cultured to about 90% confluence using conventional methods. After trypsin digestion, the cells were resuspended in cell culture medium to 4×10⁵ cells/ml and 50 µl/well were added to two 96-well cell culture plates. After incubation at 37°C for 48 h, the supernatant was aspirated and fresh culture medium was added. According to the manufacturer's method, four plasmids, pcDNA3.1(+)/CD122 (constructed by Nanjing Novoacine Biotechnology Co., Ltd, Uniprot P14784 1Met-551Val was constructed into plasmid pcDNA3.1(+)), pcDNA3.1/puro/CD132 (constructed by Nanjing Novoacine Biotechnology Co., Ltd, Uniprot P31785 1Met-551Thr was constructed into plasmid pcDNA3.1/zeo), pCMV/JAK3 (Sino Biological), and PGL4.52/STAT5RE (Promega) were transiently transfected into 293EC18 cells using a cationic transfection reagent (ExFect Transfection Reagent, Vazyme). The obtained cells were named 293EC18/βγ/JAK3/STAT5 cells. Alternatively, five plasmids, pcDNA3.1/zeo/CD25 (constructed by Nanjing Novoacine Biotechnology Co., Ltd, Uniprot P01589 1Met-551Ile constructed into plasmid pcDNA3.1/zeo), pcDNA3.1(+)/CD122 (constructed by Nanjing Novoacine Biotechnology Co., Ltd, Uniprot P14784 1Met-551Val constructed into plasmid pcDNA3.1(+)), pcDNA3.1/puro/CD132 (constructed by Nanjing Novoacine Biotechnology Co., Ltd, UniprotP31785 1Met-551Thr constructed into plasmid pcDNA3.1/zeo), pCMV/JAK3 (Sino Biological), and PGL4.52/STAT5RE (Promega) were transiently transferred into 293EC18 cells, and the obtained cells were named 293EC18/αβγ/JAK3/STAT5 cells. The transfected cells were incubated at 37°C and 5% CO₂ for another 6 h.

### 2. Test of the activation effect of each IL2 mutant on βγ and αβγ.

Each IL2 mutant was diluted 4-fold in a gradient manner using PBS buffer, with a total of 11 dilutions and a starting concentration of 65.2 nM. In the above cell culture plates, 50 µl/well of each of the serially diluted IL2 mutant samples was added in duplicate. Then, the cell culture plate was placed in an incubator at 37°C and 5% CO₂ for overnight culture. The cell culture plates were kept at room temperature for 20 ± 5 minutes to equilibrate the plates to room temperature. Bio-Lite Luciferase Assay System reagents that have been mixed and equilibrated to room temperature were added to the cell culture plates to be tested at 100 µl/well, placed on a microplate shaker at 450 rpm to mix for 2 minutes, then allowed to stand at room temperature for 3 minutes, and the chemiluminescence value was detected with an enzyme plate reader.

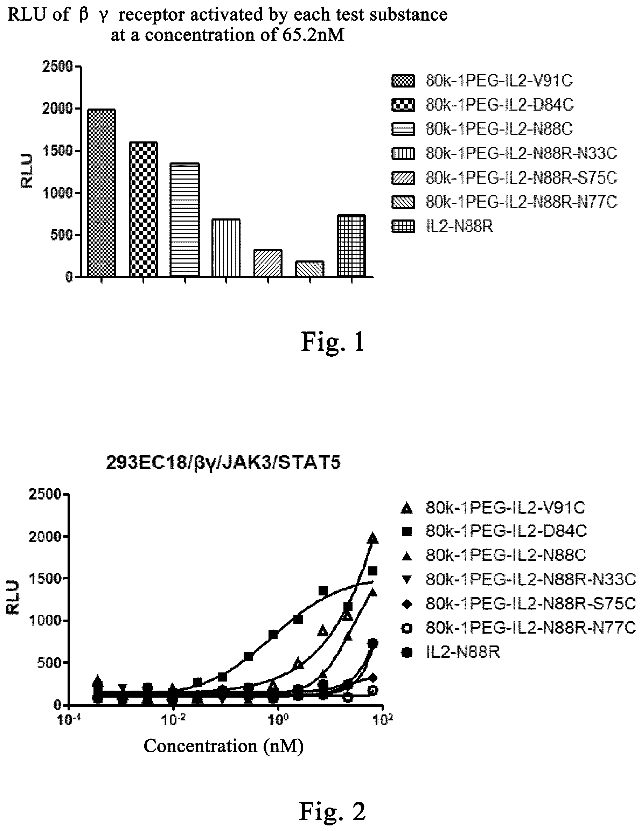

The logarithm of the final concentration of each IL-2 mutant sample was used as the X-axis, and the chemiluminescence detection results were used as the Y-axis for plotting. Data were analyzed using the four-parameter logistic regression model in GraphPad Prism 9. The results are shown in Figures 1-3 and Table 2.

**Table 2 EC50 values for the activation of the IL-2αβγ triple receptor by PEG-IL-2 mutants as determined by a reporter gene assay.**

| Test substance | EC₅₀ for activation of the IL-2αβγ triple receptor (nM) |
|---|---|
| 80k-1PEG-IL2-V91C | 0.05866 |
| 80k-1PEG-IL2-D84C | 0.003779 |
| 80k-1PEG-IL2-N88C | 0.118 |
| 80k-1PEG-IL2-N88R-N33C | 0.003864 |
| 80k-1PEG-IL2-N88R-S75C | 1.262 |
| 80k-1PEG-IL2-N88R-N77C | 0.1844 |
| IL2-N88R | 0.01663 |

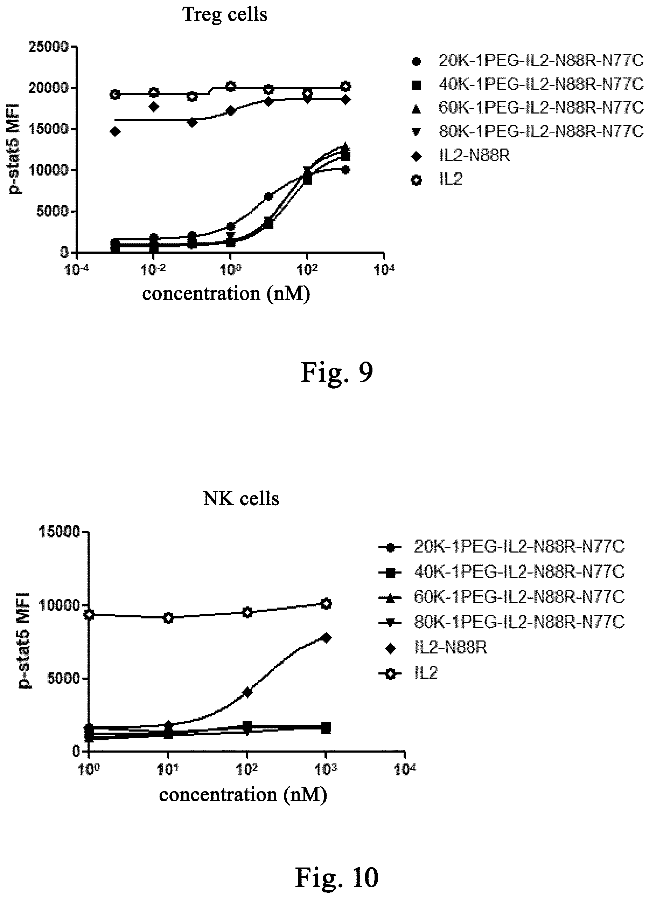

Although the N88R single-point mutation disclosed in the prior art significantly reduces the ability to activate Teff cells, it still retains relatively strong ability to activate NK cells, posing a potential safety risk. This has also been confirmed in the present application, e.g., see the results shown in Figure 10.

The results of Figure 1 show that among all the tested substances, 80k-1PEG-IL2-N88R-N33C, 80k-1PEG-IL2-N88R-S75C, and 80k-1PEG-IL2-N88R-N77C all had lower activation effects on the IL-2Rβγ dual receptor at a concentration of 65.2 nM than the activation effect of the control IL-2 N88R without PEG modification, especially, 80k-PEG-IL2-N88R-N77C exhibited the most significant reduction in activation ability. The results of Figure 2 show that the activation effects of the mutants on the IL-2Rβγ dual receptor at all concentrations were reduced, significantly lower than that of the control IL-2 N88R without PEG modification, suggesting that 80k-1PEG-IL2-N88R-N33C, 80k-1PEG-IL2-N88R-S75C, and 80k-1PEG-IL2-N88R-N77C further reduced the affinity to IL-2Rβγ on the basis of IL-2 N88R, among which 80k-1PEG-IL2-N88R-N77C exhibited the most significant reduction in activation ability.

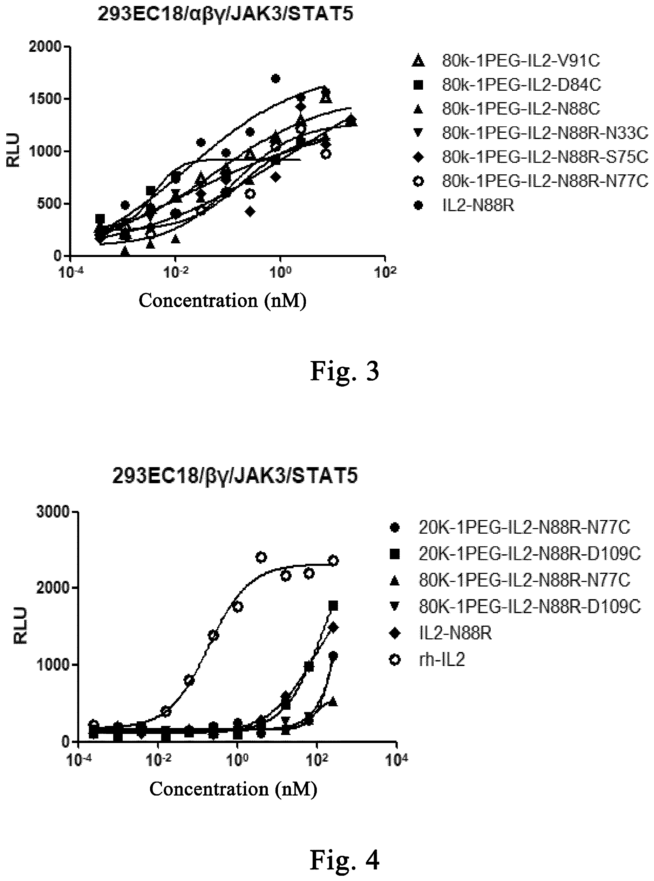

The results of Table 2 and Figure 3 show that compared with the control IL2-N88R without PEG modification, all the PEG-modified IL-2 mutants obtained in the present application retained good activation ability toward the IL-2Rαβγ triple receptor. In particular, 80k-1PEG-IL2-N88R-N33C and 80k-1PEG-IL2-D84C exhibited even stronger activation of the IL-2Rαβγ triple receptor than IL-2 N88R.

Analysis suggests that the PEGylated IL-2 mutants (80k-1PEG-IL2-N88R-N33C, 80k-1PEG-IL2-N88R-S75C and 80k-1PEG-IL2-N88R-N77C) have better retained the affinity for the IL-2Rαβγ triple receptors while minimizing the affinity for the IL-2Rβγ dual receptors, suggesting that they have a greater preference for T cells, that is, they prefer to stimulate Treg cells *in vivo* and avoid the activation effect on effector T cells (Teffs) to a greater extent.

### Example 4. Effect of polyethylene glycol modification with different molecular weights on the receptor binding preference of IL-2 mutants

According to the method of Example 2, IL2 mutants modified with PEGs of different molecular weights were prepared and purified, as shown in Table 3. For the naming convention, see Example 2:

**Table 3 information of test substance PEG-IL2 mutants**

| PEG-IL2 mutant | IL2 mutation site | Conjugation site | PEG molecular weight |
|---|---|---|---|
| 20K-1PEG-IL2-N88R-D 109C | N88R, D109C | 109 | 20kD |
| 80K-1PEG-IL2-N88R-D109C | N88R, D109C | 109 | 80kD |
| 20K-1PEG-IL2-N88R-N77C | N88R, N77C | 77 | 20kD |
| 80K-1PEG-IL2-N88R-N77C | N88R, N77C | 77 | 80kD |

According to the method of Example 3, each polyethylene glycol-modified mutant obtained in this example was assessed for the binding preference to the IL2 receptors by a reporter assay. The results are shown in Figures 4-6 and Table 4.

**Table 4 EC50 values for the activation of the IL-2αβγ triple receptor by PEG-IL-2 mutants as determined by a reporter gene assay.**

| Numbering | EC₅₀ for activation of the IL-2αβγ triple receptor (nM) |
|---|---|
| 20K-1PEG-IL2 - N88R-D109C | 0.07864 |
| 20K-1PEG-IL2-N88R-N77C | 0.07053 |
| 80K-1PEG-IL2 - N88R-D109C | 0.4067 |
| 80K-1PEG-IL2-N88R-N77C | 0.2458 |
| IL2-N88R | 0.05639 |
| rh IL-2 | 0.005428 |

The results of Figures 4-5 show that PEG-IL2-N88R-N77C had the lowest binding affinity to the IL-2Rβγ dual receptor and therefore had the lowest activation effect; PEG-IL2-N88R-D109C had activation effect comparable to that of the control IL2-N88R, and both were higher than that of PEG-IL2-N88R-N77C. This suggests that additional substitution of cysteine at amino acid position 77 of IL-2 followed by PEG modification further reduced the affinity for βγ compared with the control IL2-N88R.

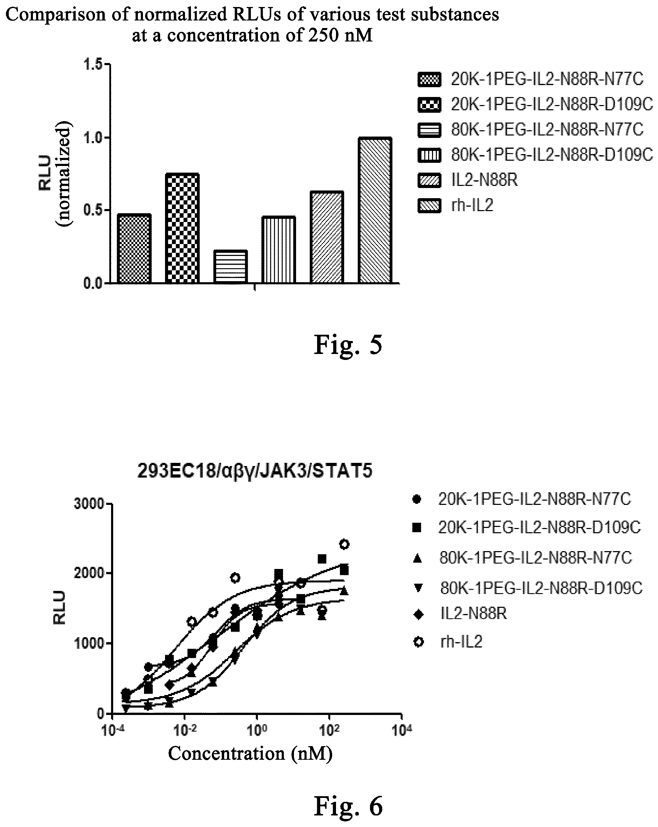

The results of Figure 6 show that, compared to the control IL-2 N88R, IL-2 variants with additional mutation of N77C or D109C and modification at the corresponding site with PEGs of different molecular weights substantially retained their affinity for the IL-2Rαβγ triple receptor, with activation effects comparable to that of the control. In addition, when modified with 80k PEG, the mutant 80K-1PEG-IL2-N88R-N77C had slightly higher activation activity than 80K-1PEG-IL2-N88R-D109C, retaining the preference for Treg cells to a greater extent.

The above studies show that PEG-IL2-N88R-N77C had significant advantages in further reducing the affinity of IL-2 for the IL-2Rβγ dual receptor and maintaining better affinity for IL-2Rαβγ triple receptor. Therefore, PEG-IL2-N88R-N77C, as a candidate, was used in the following experiments.

### Example 5. Effects of 80K-1PEG-IL2-N88R-D109C and 80K-1PEG-IL2-N88R-N77C on mice

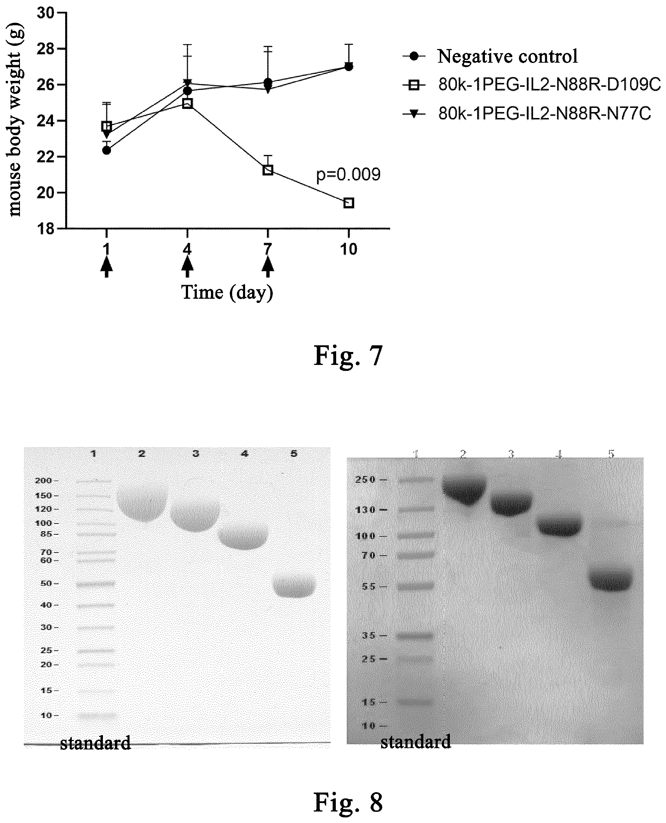

Balb/c mice were used to investigate the effects of 80K-1PEG-IL2-N88R-D109C and 80K-1PEG-IL2-N88R-N77C on the body weight and physiological characteristics of mice. SPF grade Balb/c mice were purchased, with 3 mice in each group. Administration was started on the day of grouping. The test substance or PBS (negative control group) was administered subcutaneously at a dose of 1 mg/kg, once every three days, for a total of three times. The mice were weighed before each administration and three days after the third administration . The results are shown in Figure 7. The body weight of mice in the 80K-1PEG-IL2-N88R-D109C group began to decrease after the second dose and was significantly lower than that of the negative control three days after the third dose (p=0.009), while the body weight in the 80K-1PEG-IL2-N88R-N77C group was not significantly different from that of the negative control. In addition, visual observation revealed that the mice in the 80K-1PEG-IL2-N88R-D109C-treated group were listless, had poor motility, often curled up and motionless , and had fluffy hair, while the mice in the 80K-1PEG-IL2-N88R-N77C-treated group were in good condition, similar to that of the negative control mice, with normal motility and smooth and shiny hair. The experimental results show that 80K-1PEG-IL2-N88R-N77C had less toxic side effects, while 80K-1PEG-IL2-N88R-D109C had relatively obvious toxic side effects on mice.

### Example 6. Construction of PEG-IL2-N88R-N77C mutants modified with PEGs of different molecular weights

According to the methods of Examples 1 and 2, PEGs of different molecular weights (20 kDa, 40 kDa, 60 kDa, and 80 kDa) were prepared to modify the mutant IL2-N88R-N77C, that is, PEGs of different molecular weights were conjugated to the cysteine at position 77 of the mutant. The results are shown in Figure 8, and it can be seen that the molecules of 80K-1PEG-IL2-N88R-N77C, 60K-1PEG-IL2-N88R-N77C, 40K-1PEG-IL2-N88R-N77C and 20K-1PEG-IL2-N88R-N77C were successfully prepared.

The IL2-N88R-N77C mutation sites were identified using LC-MS as follows: IL-2 and IL2-N88R-N77C were digested with trypsin, the resulting peptide fragments were analyzed by LC-MS, and the mutation site was determined by comparing the changes in the characteristic peptide fragments. The results are shown in Table 5. It can be seen that characteristic peptide fragments containing the mutated amino acid were detected in IL2-N88R-N77C, while those containing the pre-mutation amino acid were not detected.

**Table 5: Analysis of characteristic peptide fragments of IL2 and IL2 mutants**

| Sample | Peptide fragments | Theoretical mass-to-nuclear ratio (m//z, [M+2H]²⁺) | Measured mass-to-nuclear ratio (m//z, [M+2H]²⁺) | Retention time (min) |
|---|---|---|---|---|
| IL2-N88R-N77C | C₇₇FHLRPR | 493.76 | 493.51 | 22.785 |
| | DLISR₈₈ | 302.68 | 302.35 | 23.558 |
| IL2 | N₇₇FBLRPR | 470.76 | 470.64 | 15.96 |
| | DLISN₈₈INVIVLELK | 792.47 | 792.39 | 28.411 |

The PEG conjugation sites of 60K-1PEG-N88R-N77C and 80K-1PEG-N88R-N77C were analyzed using LC-MS as follows: IL2-N88R-N77C and PEG-IL2-N88R-N77C were digested with trypsin, and the resulting peptide fragments were analyzed by LC-MS. By comparing the decrease of specific peptide fragments before and after PEGylation, the PEG conjugation sites were identified. The results are shown in Table 6. It can be seen that the C₇₇FHLRPR peptide fragment in the PEG conjugate is reduced to less than 5% of IL2-N88R-N77C, indicating that the PEG conjugation site was on the cysteine at position 77 of the IL-2 mutant, and the site-specific modification ratio is greater than 95%.

**Table 6: Analysis of the ratio of PEG conjugate and IL2 mutant protein characteristic peptide fragment (C₇₇FHLRPR)**

| Sample | Ion current peak area | The ratio to the peak area of the unmodified protein | C77 site-specific conjugation ratio |
|---|---|---|---|
| 60K-1PEG-IL2-N88R-N77C | 2601001 | 4.5% | 95.5% |
| 80K-1PEG-IL2-N88R-N77C | 1458660 | 2.5% | 97.5% |
| IL2-N88R-N77C | 57993100 | / | / |

### Example 7. Activation of P-STAT5 in different cell subsets of human PBMCs by IL-2 (N88R-N77C) mutants modified with PEGs of different molecular weights

In the example, the activation effect of each mutant prepared in Example 6 on P-STAT5 in different cell subsets (Tregs, CD8+T cells, NK cells) from human PBMCs (Miaoshun Biotechnology) was detected.

### Tregs and CD8⁺ T cells

Human PBMC cells were thawed and resuspended to 1.5×10⁶ cells/ml in RPMI1640 medium containing 10% FBS. 100 µl was added to each well of a V-bottom 96-well culture plate. Each IL-2 mutant prepared in Example 6 was diluted 10-fold in series: 1000 nM was used as the working concentration for the first well, and then 100 µl/well of each serial dilution was added to each cultured cell, stimulated at 37°C for 30 min, and the reaction was terminated on ice. The cells were treated with a commercial fixative and permeabilization agent (BD biosciences 558049 & 558050), and stained with antibodies against CD3, CD4, CD25, and CD8 according to conventional methods. **P-STAT5** antibody staining was also performed (Table 7) to detect the mean fluorescence intensity (MFI) of **P-STAT5** in CD3+/CD4+/CD25+ (Treg cells) and CD8+/CD3+ cells (CD8+ T cells⁺ T cells) by flow cytometry.

### NK cells

Human PBMCs were thawed and NK cells were isolated using a commercial kit (Miltenyi Biotec, 130-092-657) and LS separation column (Miltenyi Biotec, 130-042-401). The cells were resuspended to 2.5×10⁵ /ml in RPMI1640 complete medium and added to a 96-well plate at 25,000 cells/well. The proportion of CD3⁻CD56⁺ cells in the isolated cells was detected by flow cytometry to detect the efficiency of cell purification. RPMI1640 complete medium was used to prepare 10-fold gradient dilution solutions (1000 nM-1 nM) of each IL-2 mutant prepared in Example 5, and then each serial dilution was added to each cultured cell well at 100 µl/well in duplicate. After stimulating the cells at 37°C for 30 minutes, the cells were treated with a commercial fixative and permeabilization agent (BD biosciences 558049 & 558050), stained with **P-STAT** 5 antibody, and the mean fluorescence intensity (MFI) of cellular **P-STAT** 5 was detected.

The experimental results are shown in Figures 9-11: IL-2-N88R-N77Cs modified with PEGs of different molecular weights had similar behavior in activating Tregs in PBMCs, and had no activation effect on NK and CD8⁺ T. Table 8 shows that the IL-2-N88R-N77C mutant modified with 20 kDa PEG exhibited a slightly lower EC₅₀ for P-STAT5 activation in Treg cells compared to the EC₅₀ values obtained by IL-2-N88R-N77C mutant modified with other PEG molecular weights. Furthermore, IL-2-N88R-N77C mutants modified with 40-80 k PEGs resulted in a comparable level of Treg activation.

**Table 7 List of labeled antibodies for human PBMCs by flow cytometry**

| Labeled Antibodies | Source | Catalog Number |
|---|---|---|
| PE mouse- Anti-human CD3 | BD Biosciences | 552127 |
| BB700 mouse Anti-human CD4 | BD Biosciences | 566392 |
| FITC Anti-human CD8a | Invitrogen | 11-0088-42 |
| BB515 mouse Anti-human CD25 | BD Biosciences | 564467 |
| FITC anti-human CD16 | BioLegend | 302005 |
| Alex Fluor 647 mouse anti-human p-stat5 | BD Biosciences | 562076 |

**Table 8 EC₅₀ of human Treg cell activated by IL2-N88R-N77C modified with PEGs of different molecular weights**

| Mutant name | EC₅₀ for Activation of Treg (nM) |
|---|---|
| 20K-1PEG-IL2-N88R-N77C | 6.138 |
| 40K-1PEG-IL2-N88R-N77C | 37.99 |
| 60K-1PEG-IL2-N88R-N77C | 35.06 |
| 80K-1PEG-IL2-N88R-N77C | 29.75 |

### Example 8. Pharmacodynamic (PD) study of PEG-IL2-N88R-N77C in mice

In this example, the pharmacodynamics (PD) of each IL-2 mutant obtained in Example 6 was studied in mice after a single administration to mice. SPF grade Balb/c mice were purchased and randomly divided into groups according to body weight, with 3 mice in each group. Drug administration started on the day of grouping. The dosing and grouping information is shown in Table 9, where G1-G5 were administered once, G6 and G7 were administered once a day, for a total of three doses. 30 µl of blood was taken from each group of mice, for PEGylated IL2 mutants and the negative control, blood was collected on day 0 (pre-administration), day 2, 3, 4, 5, 6 and 7 (only for G3, G4 and G5), and for IL2 and IL2-N88R, blood was collected on day 0 (pre-administration), day 1, 2 and 3. The number of each cell subpopulation was measured by flow cytometry. Antibodies against the staining marker used in flow cytometry are shown in Table 10, and the gating settings are shown in Table 11.

**Table 9 Dosing and grouping table for single-dose PD study in mice**

| Group | Drug | Dosage (mg/kg) | Route of administration |
|---|---|---|---|
| G1 | 20K-1PEG-IL2-N88R-N77C | 1 | s.c. |
| G2 | 40K-1PEG-IL2-N88R-N77C | 1 | s.c. |
| G3 | 60K-1PEG-IL2-N88R-N77C | 1 | s.c. |
| G4 | 80K-1PEG-IL2-N88R-N77C | 1 | s.c. |
| G5 | Negative control | / | s.c. |
| G6 | IL2 | 1 | i.v. |
| G7 | IL2-N88R | 1 | i.v. |

**Table 10 List of labeled antibodies for single-dose PD study in mice by flow cytometry**

| Labeled Antibodies | Source | Catalog Number |
|---|---|---|
| FITC Rat Anti-Mouse CD3 Molecular Complex Clone 17A2(RUO) | BD Biosciences | 555274 |
| PerCP-Cy^{™}5.5 Rat Anti-Mouse CD4 Clone RM4-5 (RUO) | BD Biosciences | 550954 |
| APC Rat Anti-Mouse CD8a Clone 53-6.7 (RUO) | BD Biosciences | 553035 |
| PE Rat Anti-Mouse CD25 Clone PC61 (RUO) | BD Biosciences | 553866 |
| Anti-mouse CD49b | BD Biosciences | 560628 |

**Table 11 Gating parameters for single-dose PD study in mice by flow cytometry**

| Cell Type | Marker profile |
|---|---|
| CD8⁺ T cells | CD3+ CD8+ |
| Treg cells | CD3+CD4+ CD25+ |
| NK cells | CD3-CD49b+ |

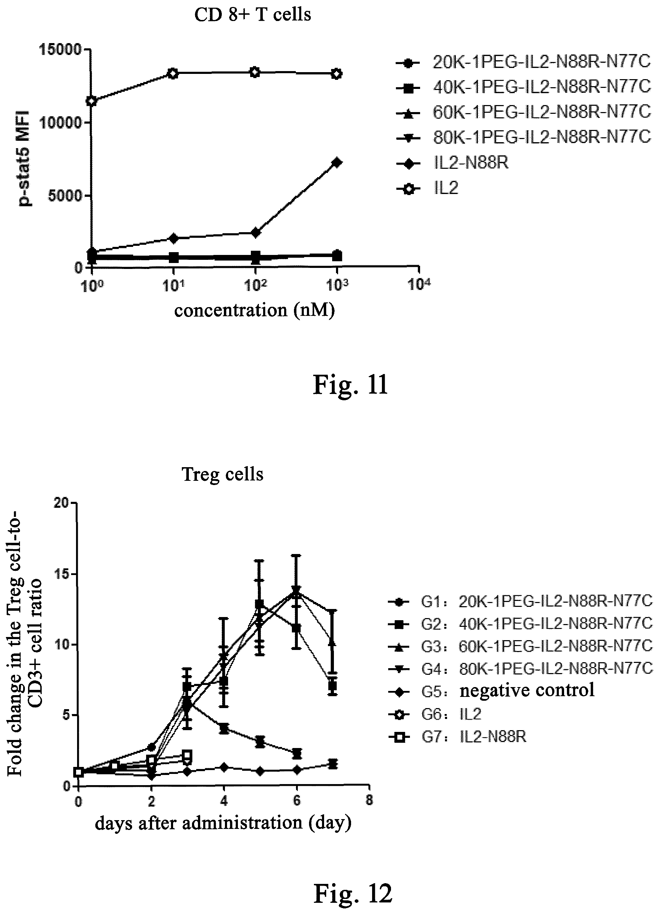

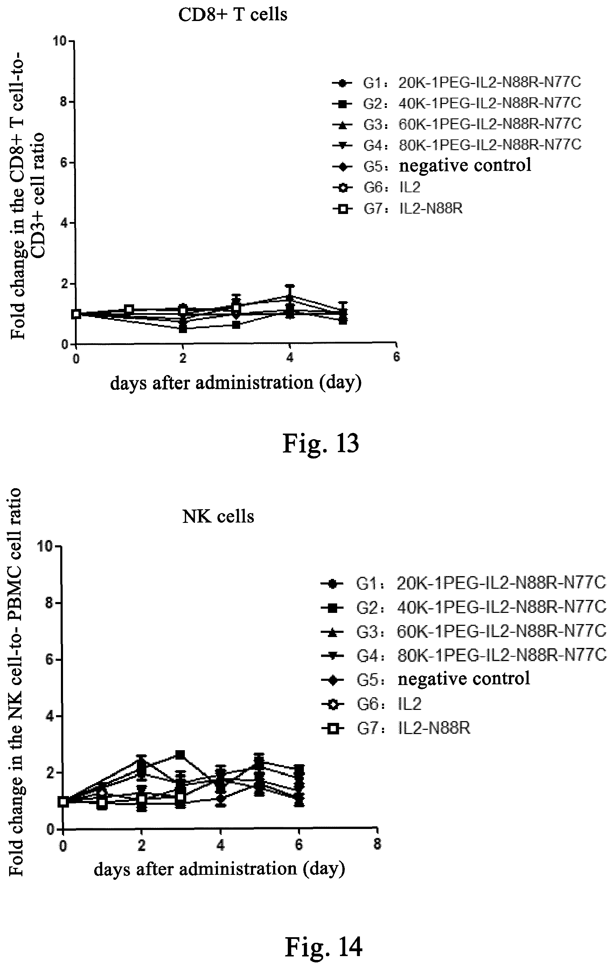

The experimental results are shown in Figures 12-14: all PEGylated IL-2 mutants (Groups G1, G2, G3, and G4) at a dose of 1 mg/kg significantly activated Treg cells, as indicated by an increase in the proportion of Treg cells. With increasing PEG molecular weight, both the timing and magnitude of the peak of Treg cell proportion increased. No significant difference was observed between the 60K-1PEG-IL2-N88R-N77C (Group G3) and the 80K-1PEG-IL2-N88R-N77C (Group G4), both reaching activation peak on Day 6, with up to about 15-fold increase in Treg proportion, substantially higher than that observed in the IL-2 group (Group G6) and the IL-2-N88R dosing group (Group G7) (Figure 12). In contrast, none of the tested PEGylated IL-2 mutants showed notable activation of CD8⁺ T cells or NK cells (Figures 13 and 14).

### Example 9. Pharmacokinetic study of PEG-IL2-N88R-N77C in mice

In this example, the pharmacokinetics (PK) of various IL-2 mutants modified with PEGs of different molecular weights obtained in Example 5 in mice was detected after a single administration to mice. Balb/c mice were purchased, with 4 mice in each group. Each IL-2 mutant to be tested was administered subcutaneously once at a dose of 1 mg/kg. Blood was collected from the orbit at different time points after administration, and the plasma was separated and the plasma drug concentration was detected by ELISA. The main pharmacokinetic parameters were calculated, and C-T curve was plotted.

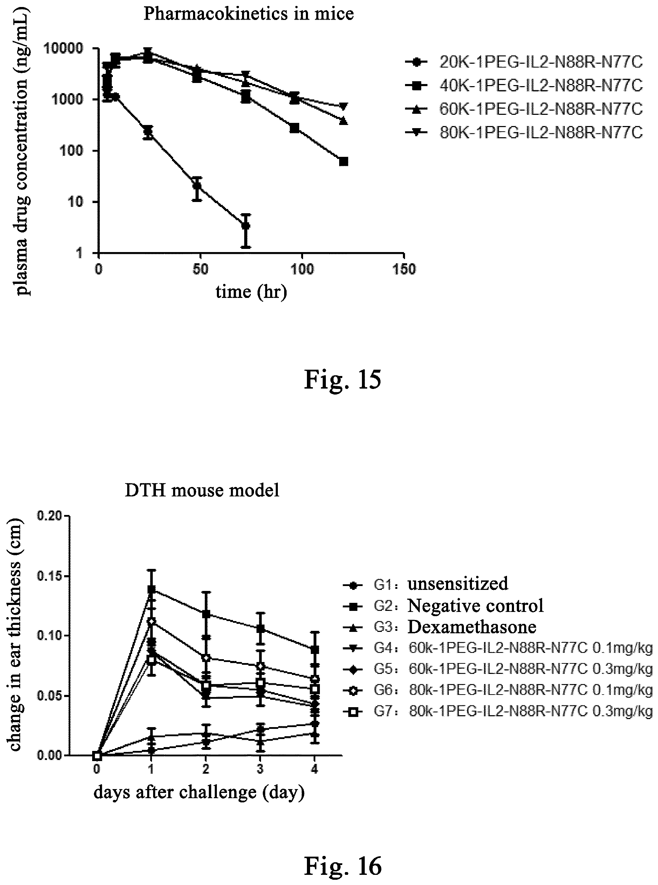

The results are shown in Figure 15 and Table 12. With increasing the molecular weight of PEG to modify the IL-2 mutants, the drug metabolism rate of the corresponding mutant gradually decreased. The half-life of 60K and 80K modified products was significantly longer than that of 20K and 40K modified products, and the drug exposure was increased. Modification with polyethylene glycol of 40K and higher molecular weight could significantly reduce the clearance (CL) of IL2 in mice. The mean half-lives of the 60K- and 80K-modified products were 20.78 hours and 25.23 hours, respectively, which were approximately 2.7-3.3 times longer than those of the 20K- and 40K-modified products. The mean residence times (MRT) of the 60K- and 80K-modified products were 39.45 hours and 40.08 hours, respectively, showing similar values. The pharmacokinetic data indicate that the 60K and 80K exhibited more favorable *in vivo* metabolic profiles, supporting extended dosing intervals and reduced dosing frequency.

The key pharmacokinetic parameters are summarized as follows:

**Table 12 Pharmacokinetic parameters of a single administration in mice**

| Test substance | AUC inf (h.ng/mL) | AUClast (h.ng/mL) | Cmax (ng/mL) | T_{1/2} (h) | MRT (h) | CL (mL/h) | Tmax (h) |
|---|---|---|---|---|---|---|---|
| 20K-1PEG-IL2-N88R-N77C | 22882.36±3522.54 | 21762.28±3586.39 | 1276.71±273.3 | 7.62±0.57 | 11.68±0.08 | 44.4±6.99 | 4-8 |
| 40K-1PEG-IL2-N88R-N77C | 307713.36±53891.44 | 306707.67±53987.97 | 6581.76±1168.04 | 11.33±0.97 | 31.57±1.16 | 3.32±0.5 | 8-24 |
| 60K-1PEG-IL2-N88R-N77C | 392062.52±33891.53 | 380552.49±34195.95 | 6633.54±581.69 | 20.78±1.92 | 39.45±0.44 | 2.56±0.22 | 8-24 |
| 80K-1PEG-IL2-N88R-N77C | 463630.62±37728.2 | 437859.4±41090.6 | 8418.72±491.4 | 25.23±3.45 | 40.08±2.1 | 2.17±0.18 | 24 |

### Example 10. KLH-induced delayed-type hypersensitivity (DTH) model in BALB/C mice

The inflammation-suppressing effects of 60K-1PEG-IL2-N88R-N77C and 80K-1PEG-IL2-N88R-N77C were evaluated in the delayed hypersensitivity model of Keyhole Limpet Hemocyanin (KLH)-induced ear swelling in BALB/c mice. SPF-grade Balb/c mice were used and randomly divided into groups (see Table 13 for group setting and dosing regimen). On the day of grouping, except for Group G1, the other groups were sensitized with KLH/CFA emulsion at four subcutaneous points on the abdomen. KLH/CFA emulsion was prepared by emulsifying KLH and complete Freund's adjuvant (CFA) in a 1:1 ratio. Each mouse was injected with 100 µg KLH/CFA emulsion (25 µg/point). Drug administration started on the day of sensitization. Five days later, KLH was diluted to 1 µg/µL with saline and injected intradermally into both ears of each mouse using a microsyringe, with 5 µL in each ear for challenge. The thickness of the mouse ears was measured daily using a micrometer screw from day 5 to day 9.

**Table 13 Drug administration and grouping of mouse DTH model**

| Group | Drug | Number of mice | Sensitization | Attack | Drug administration |
|---|---|---|---|---|---|
| G1 | Saline | 3 | x | √ | 0.1ml Q3d i.p. |
| G2 | Saline | 4 | √ | √ | 0.1ml Q3d i.p. |
| G3 | Dexamethasone | 3 | √ | √ | 3 mg/kg daily i.p. |
| G4 | 60K-1PEG-IL2-N88R-N77C | 3 | √ | √ | 0.1 mg/kg Q3d s.c. |
| G5 | 60K-1PEG-IL2-N88R-N77C | 4 | √ | √ | 0.3 mg/kg Q3d s.c. |
| G6 | 80K-1PEG-IL2-N88R-N77C | 3 | √ | √ | 0.1 mg/kg Q3d s.c. |
| G7 | 80K-1PEG-IL2-N88R-N77C | 3 | √ | √ | 0.3 mg/kg Q3d s.c. |

After KLH challenge, the ear thickness of unsensitized mice in the G1 group did not show a significant increase, while the other groups exhibited ear swelling, among which, the negative control group (G2) reached a peak in ear thickness at 24 hours. The PEGylated IL-2 mutant drug groups (G4, G5, G6, and G7) significantly inhibited the increase in ear thickness in a dose-dependent manner, indicating that the tested drugs can suppress the inflammatory response in the DTH mouse model (Figure 16).

### Example 11. Pharmacodynamic and pharmacokinetic studies of PEG-IL2-N88R-N77C in cynomolgus monkeys

In this study, 60K-1PEG-IL2-N88R-N77C and 80K-1PEG-IL2-N88R-N77C were subcutaneously administered to cynomolgus monkeys (two in each group, one male and one female). Blood was collected before administration and at different time points after administration and the effects on the following immune cells were evaluated by flow cytometry. For information on administration and grouping, see Table 14. Blood was collected before and after administration (4h, 8h, 24h, 48h, 72h, 120h, 168h, 240h, 336h) in the first cycle and after administration (8h, 24h, 48h, 72h, 120h, 168h, 240h) in the second cycle to detect plasma drug concentration. The gating settings used in flow cytometry are shown in Table 15. The pharmacokinetic parameters were analyzed using software and the results are shown in Tables 16 and 17.

**Table 14 Dosing schedule for PK and PD studies in cynomolgus monkeys**

| Drug | Number of cynomolgus monkeys | First-cycle dosing | Second-cycle dosing |
|---|---|---|---|
| 60K-1PEG-IL2-N88R-N77C | 2 (1M+1F) | Day 1: 50 µg/kg | Day 15: 400 µg/kg |
| 80K-1PEG-IL2-N88R-N77C | 2 (1M+1F) | Day 1: 50 µg/kg. | Day 15: 400 µg/kg. |

**Table 15 Flow cytometry gates for PD study in cynomolgus monkey**

| Cell Type | Marker profile |
|---|---|
| Treg | CD45+CD3+/CD4+/CD25+/Foxp3+ |
| CD8⁺ T | CD45+/CD3+/CD8+ |
| NK | CD45+/CD3-/CD56+ |

**Table 16 Pharmacokinetic parameters of the first-cycle dosing in cynomolgus monkeys**

| Test substance | AUC inf (h.ng/mL) | AUClast (h.ng/mL) | Cmax (ng/mL) | T_{1/2} (h) | MRT (h) | CL (mL/h) | Tmax (h) |
|---|---|---|---|---|---|---|---|
| 60K-1PEG-IL2-N88R-N77C-female | 181709.8 | 148610.9 | 701.0718 | 129.34 | 128.4211 | 0.28 | 72 |
| 60K-IPEG-IL2-N88R-N77C-male | 213003.1 | 157799.2 | 815.2866 | 166.9766 | 135.9233 | 0.23 | 48 |
| 80K-1PEG-IL2-N88R-N77C-female | 186055.6 | 147924.8 | 902.5692 | 145.8838 | 122.9092 | 0.27 | 8 |
| 80K-1PEG-IL2-N88R-N77C-male | 278194.8 | 184757.5 | 824.4732 | 216.1721 | 142.9087 | 0.18 | 48 |

**Table 17 Pharmacokinetic parameters of the second-cycle dosing in cynomolgus monkeys**

| Test substance | AUC inf (h.ng/mL) | AUClast (h.ng/mL) | Cmax (ng/mL) | T_{1/2} (h) | MRT (h) | CL (mL/h) | Tmax (h) |
|---|---|---|---|---|---|---|---|
| 60K-1PEG-IL2-N88R-N77C-female | 2158472 | 1360931 | 10419.65 | 174.8 | 101.9 | 0.19 | 8 |
| 60K-IPEG-IL2-N88R-N77C-male | 2122205 | 1412112 | 10199.77 | 148.1 | 100.7 | 0.19 | 8 |
| 80K-1PEG-IL2-N88R-N77C-female | 2286053 | 1481823 | 8785.158 | 156.4 | 103.1 | 0.17 | 8 |
| 80K-1PEG-IL2-N88R-N77C-male | 2635620 | 1885448 | 11432.93 | 133.6 | 102.1 | 0.15 | 48 |

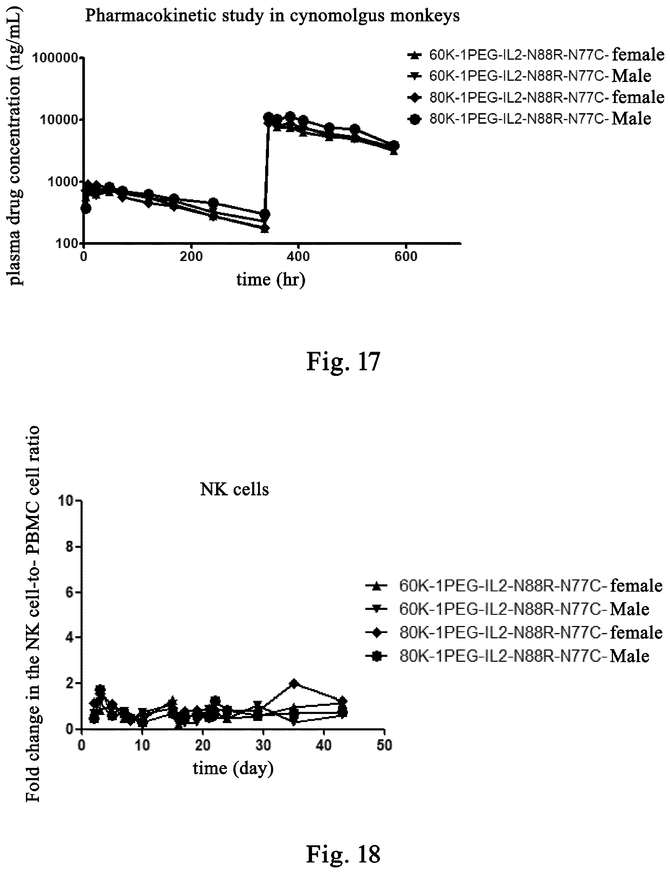

The results of the PK study show (Figure 17) that the two test substances had similar half-lives and MRTs after two consecutive subcutaneous administrations (Q2W) in cynomolgus monkeys. The half-life of 60K-1PEG-IL2-N88R-N77C in monkeys ranged from 129.3 to 167.0 hours, and that of 80K-1PEG-IL2-N88R-N77C ranged from 133.6 to 216.2 hours, which is significantly longer than the half-life of IL-2 or IL-2-Fc fusion proteins reported in the literature. The primary reason is that the average molecular weight of the test substance is higher than 70kD, larger than the glomerular filtration molecular weight limit, thereby reducing renal clearance and significantly extending the half-life.

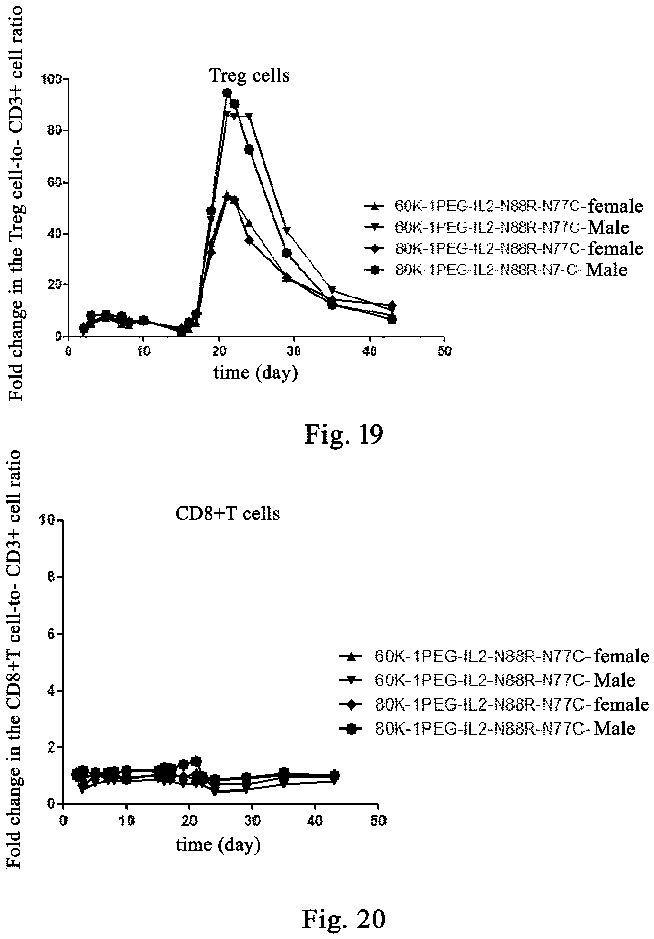

The results of the PD study showed that the tested PEG-modified IL-2 mutants prepared in the present application significantly stimulated the increase in the number of peripheral Treg cells in cynomolgus monkeys at a dose of 400 µg/kg, with the maximum fold increase exceeding 90-fold relative to the pre-administration baseline and an average peak of 75-fold (Figure 19). Three weeks after administration, the average fold increase in Treg cells remained at approximately 14-fold, demonstrating sustained and potent Treg cell-stimulating activity in monkeys. The detection of NK cells and CD8 T cells indicated that, despite the strong expansion of Treg cells, the PEG-modified IL-2 mutants prepared in the application did not induce significant changes in the numbers of NK cells or CD8+ T cells (Figures 18 and 20), reflecting its optimized preference for Treg cell activation.

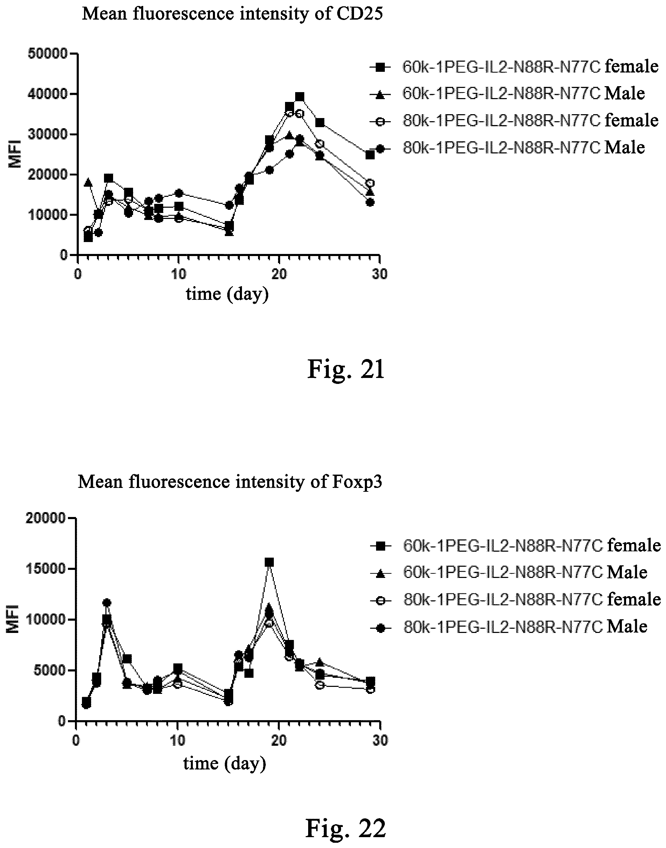

Analysis of the mean fluorescence intensity of CD25 and Foxp3 proteins on Treg cells at various time points (Figures 21 and 22) showed increased expression intensity of CD25 and Foxp3 after dosing cycle. CD25 peak appeared on day 3 after the first round of administration. The increase in CD25 after the second round of administration was significantly higher than that of the first round, and the peak appeared on day 7 after administration. Foxp3 peaks appeared around day 4 after the first and second rounds of administration. The above data indicate that the peripheral Treg cells expanded after administration exhibited increased expression of both CD25 and Foxp3.

### Example 12. Preliminary safety study of PEG-IL2-N88R-N77C in cynomolgus monkeys

In this study, 60K-1PEG-IL2-N88R-N77C and 80K-1PEG-IL2-N88R-N77C were subcutaneously administered to cynomolgus monkeys (two in each group, one male and one female). The dosing and grouping information is shown in Table 18. A 3-week recovery period was set at the end of the experiment.

**Table 18 Dosing schedule for preliminary safety study in cynomolgus monkeys**

| Drug | Number of cynomolgus monkeys | First-cycle dosing | Second-cycle dosing | Third-cycle dosing |
|---|---|---|---|---|
| 60K-1PEG-IL2-N88R-N77C | 2 (1M+1F) | Day 1: 50 µg/kg | Day 15: 400 µg/kg | Day 43: 1200 µg/kg |
| 80K-1PEG-IL2-N88R-N77C | 2 (1M+1F) | Day 1: 50 µg/kg. | Day 15: 400 µg/kg. | Day 43: 1200 µg/kg |

All animals in each group survived under the above dosages and cycles, and no obvious abnormalities were observed in body weight, body temperature, respiration, electrocardiogram and blood pressure tests. No obvious abnormalities were observed in gross observation during the dosing period at doses of 50 µg/kg and 400 µg/kg. Abnormal feces occurred at the high dose of 1200 µg/kg, and male animals in each group had skin redness (whole body/groin/hind limbs) or desquamation. The cytokines IFN-γ, TNF-α, IL-5, IL-6, and IL-10 were detected before the third administration and 24 h, 48 h, 72 h, and 120 h after the third administration, and no increase in cytokines was detected. After the recovery period, an autopsy was performed and no drug-related organ abnormalities were found.

Anti-drug antibodies (ADA) were detected in the serum of all test animals after the second and third administrations, and all samples were negative.

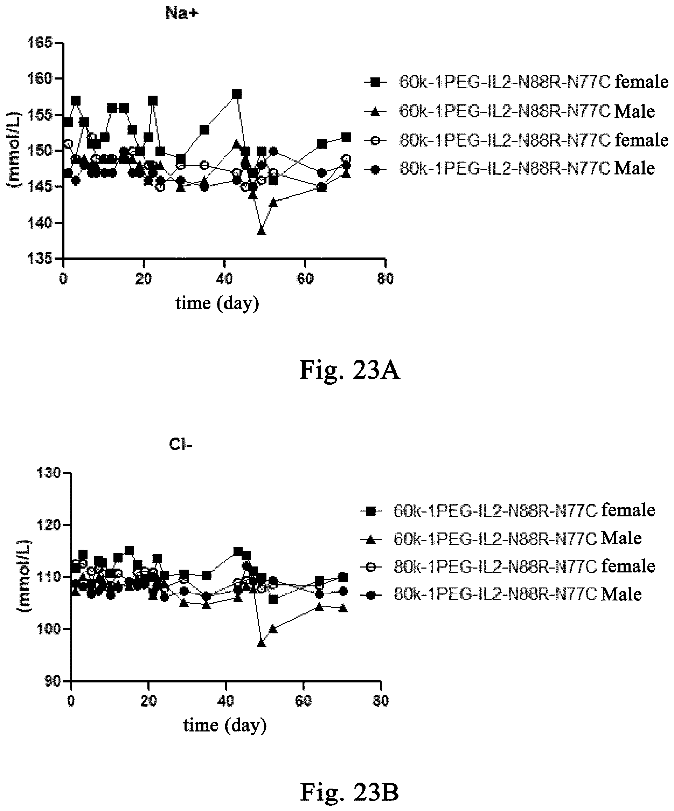

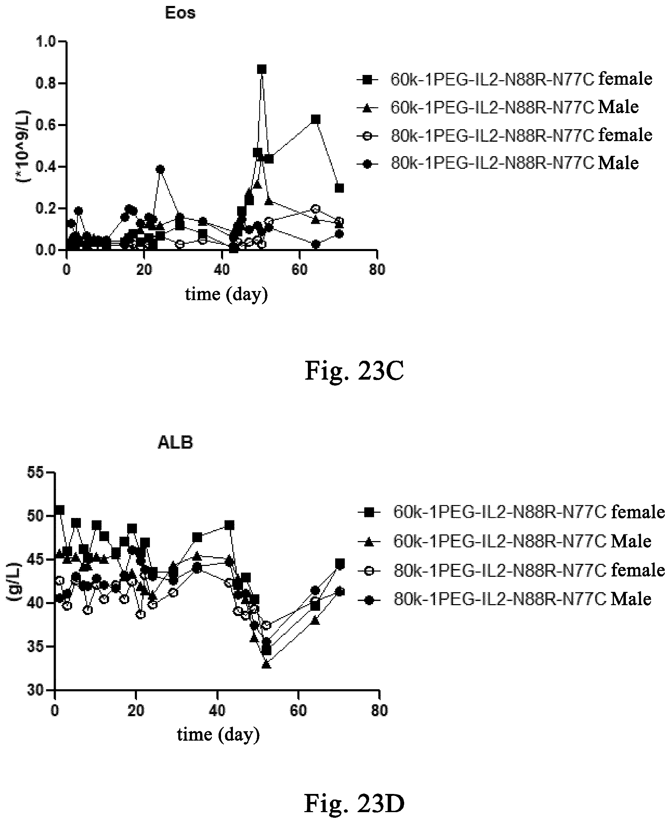

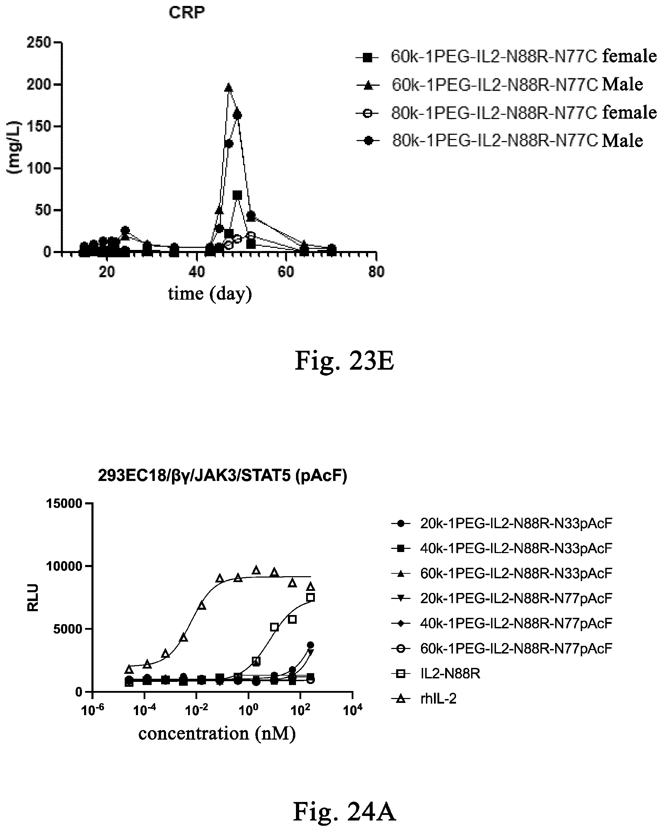

In hematology and blood biochemistry, some individual animals showed decreases in Na⁺ and Cl⁻, increases in eosinophils (EOS), a slight decrease in albumin (ALB), and a marked elevation in C-reactive protein (CRP) after the final dose (Figure 23).

The above results show that in cynomolgus monkeys, both 60k-1PEG-IL2-N88R-N77C and 80k-1PEG-IL2-N88R-N77C induced clear activation and proliferation of peripheral blood Treg cells at doses ≤400 µg/kg, with no significant toxic responses observed, suggesting reliable safety within this effective dose range. At a high dose of 1200 µg/kg, individuals showed adverse reactions similar to those known after IL-2 administration, such as diarrhea, increased white blood cells, decreased albumin, increased CRP and so on.

These data suggest that the therapeutic window for treatment of patients with 60k-1PEG-IL2-N88R-N77C and 80k-1PEG-IL2-N88R-N77C is expected to be significantly wider than that of IL-2.

### Example 13. Construction, expression and purification of interleukin-2 mutants with non-natural amino acid (phenylalanine analog) mutations

The interleukin-2 mutants with non-natural amino acid (phenylalanine analog) were obtained using the reference method (An enhanced system for unnatural amino acid mutagenesis in E. coli. J Mol Biol. 2010 Jan 15;395(2):361-74.). Based on the protein sequence of human mature IL-2 (SEQ ID NO: 1), according to the mutation sites shown in Table 19, the parental amino acid codon of each site was replaced with the amber codon TAG. cDNAs encoding the corresponding IL-2 mutants with each codon mutation shown in Table 19 were designed accordingly. An initiation codon and an NdeI restriction site were added to the 5' end of each cDNA, and a stop codon and an EcoRI restriction site were added to the 3' end. The cDNAs were synthesized by DNA synthesis and subsequently cloned into the expression vector pET30a(+). The calcium chloride method was used to co-transform BL21 (DE3) competent cells with the expression vector pET30a (+) containing the corresponding cDNA sequence and the auxiliary plasmid pEVOL-pAcF that can specifically introduce p-Acetyl-L-phenylalanine (pAcF). Positive clones were picked from the kanamycin and chloramphenicol double-resistant plates and used as expression strains for each mutant protein after sequencing verification.

**Table 19. List of IL-2 mutants**

| Mutant name | Mutation site | SEQ ID NO: |
|---|---|---|
| IL2-N88R-N33pAcF | N88R, N33pAcF | SEQ ID NO: 9 |
| IL2-N88R-N77pAcF | N88R, N77pAcF | SEQ ID NO: 10 |

The expression strains of each IL2-pAcF mutant protein were inoculated into GMML medium and cultured in a shaker at 37°C and 250 rpm until the concentration of bacterial solution reached to OD₆₀₀ of 0.6-0.8. 1 mM p-acetylphenylalanine, 0.2% arabinose and 1 mM IPTG were added to induce the expression of each mutant protein for 4 hours, and the bacterial solution was centrifuged at 13900 g to harvest the bacteria. The bacteria were washed and resuspended with 50 mM Tris-HCl, 5 mM EDTA, pH 8.0 buffer, disrupted at 700-1000 bar pressure, and the inclusion bodies were harvested by centrifugation at 13900 g.

Then, purification was performed with reference to Example 1 to obtain the purified interleukin-2 non-natural amino acid mutant.

### Example 14. Polyethylene glycol modification and purification of IL-2 mutants with non-natural amino acid mutations

The IL-2 non-natural amino acid mutant obtained in Example 13 was modified and purified according to references (CN110078814A, CN104163864B). The PEG used in this example contains a hydroxylamine group (oxyamine), which can react specifically with the p-acetylphenylalanine residue at a specific mutation site of the mutant, thereby allowing the PEG to site-specifically modify the corresponding IL-2 mutant. Specifically, the purified IL-2 mutant protein was taken, the solution was changed to 10 mM sodium acetate, 5% trehalose, pH 4.5 buffer, and the protein concentration was adjusted to 3 mg/ml, PEG-hydroxylamine (20-80k, such as 20k, 40k, 60k, 80k, Xiamen Sinopeg) was added at a molar ratio of 1:5 of IL-2 mutant: PEG, and NH₄Cl and acetylhydrazine were added thereto for catalysis, and the reaction was carried out at 2-8°C for at least 48 hours.

Then, purification was performed with reference to Example 2 to obtain the purified PEG-modified IL-2 non-natural amino acid mutant protein.

### Example 15. Binding preference of PEG-modified IL-2 mutants with different non-natural amino acids (phenylalanine analogs) to IL-2 receptor

Referring to Example 3, the phosphorylation of STAT5 was used to evaluate the ability of the PEG-modified IL-2 mutants having a non-natural amino acid mutation prepared in Example 14 to induce signal transduction via the IL-2 receptor.

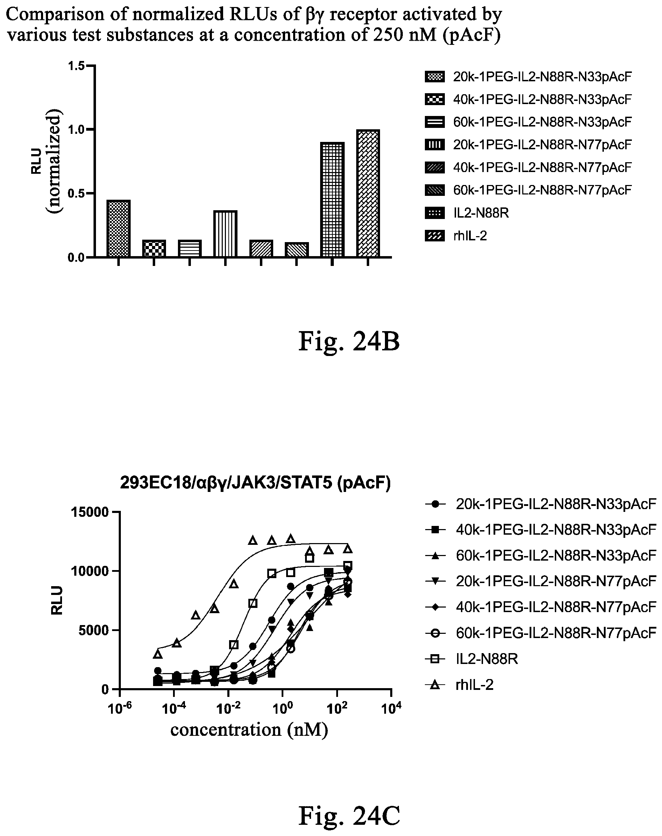

The results are shown in Figure 24 and Table 20. The analysis results show that the PEGylated IL-2 mutant with non-natural amino acid (phenylalanine analog) mutation obtained in Example 14 retained good binding affinity to the IL-2Rαβγ triple receptor, while exhibiting reduced affinity for the IL-2Rβγ dual receptor. Notably, when the PEG molecular weight exceeded 40 k, the STAT5 reporter gene signal via IL-2Rβγ dual receptor at high concentrations was reduced to 15%-30% of that induced by IL-2.This suggests an improved preference for Treg cells, that is, more preference for the *in vivo* activation of Treg cells, while avoiding the activation of effector T cells (Teffs) to a greater extent.

**Table 20 EC50 values for the activation of the IL-2αβγ triple receptor by PEG-IL-2 mutants as determined by a reporter gene assay (pAcF).**

| Test substance | Activation of IL-2αβγ triple receptor EC50 (nM) |
|---|---|
| 20k-1PEG-IL2-N88R-N33pAcF | 0.319 |
| 40k-1PEG-IL2-N88R-N33pAcF | 3.688 |
| 60k-1PEG-IL2-N88R-N33pAcF | 9.21 |
| 20k-1PEG-IL2-N88R-N77pAcF | 0.5005 |
| 40k-1PEG-IL2-N88R-N77pAcF | 1.916 |
| 60k-1PEG-IL2-N88R-N77pAcF | 5.118 |
| IL2-N88R | 0.03659 |
| rh IL-2 | 0.004105 |

### Example 16. Construction, expression and purification of interleukin-2 mutants with non-natural amino acid (lysine analog) mutations

According to the reference method (Swiderska KW, Szlachcic A, Czyrek A, Zakrzewska M, Otlewski J. Site-specific conjugation of fibroblast growth factor 2 (FGF2) based on incorporation of alkyne-reactive unnatural amino acid. Bioorg Med Chem. 2017 Jul 15;25(14):3685-3693) and the basic construction idea of Example 13, the parent amino acid at each mutation site was replaced with N-ε-propargyloxycarbonyl-L-lysine (Prk) according to the mutation sites shown in Table 20, and then the corresponding verification, expression and purification were carried out to obtain the corresponding interleukin 2 mutant with non-natural amino acid (lysine analog) mutations.

**Table 21. List of IL-2 mutants**

| Mutant name | Mutation site | SEQ ID NO: |
|---|---|---|
| IL2-N88R-N33Prk | N88R, N33Prk | SEQ ID NO: 11 |
| IL2-N88R-N77Prk | N88R, N77Prk | SEQ ID NO: 12 |

### Example 17. Polyethylene glycol modification and purification of IL-2 mutants with non-natural amino acid (lysine analog) mutations

The IL-2 mutant obtained in Example 16 was PEG-modified and purified according to reference (CN101265298). The PEG (20-80k, such as 20k, 40k, 60k, 80k, Xiamen Sinopeg) used in this example contains an azide group, which can react specifically with the alkyne group in the Prk structure of the above mutant at a specific mutation site, thereby allowing PEG to site-specifically modify the corresponding IL-2 mutant. Specifically, the IL2-Prk mutant protein was ultrafiltered into 25 mM PB, pH 6.5, with a concentration of >2.5 mg/ml. 2.5 mg of liquid-exchanged IL2-Prk mutant protein was taken, and added PEG raw material at a 10-fold molar ratio, and then added THPTA to the reaction solution to a final concentration of 1 mM, CuSO4·5H2O to a final concentration of 0.2 mM, sodium ascorbate to a final concentration of 10 mM, and aminoguanidine to a final concentration of 5 mM. The reaction was carried out at room temperature for about 3 h, and the PEG-modified protein was obtained by Macro Cap SP chromatography purification. After the polyethylene glycol modification reaction, purification was performed according to Example 2 to obtain the purified PEG-modified IL-2 mutant protein with lysine analog.

### Example 18. Binding preference of PEG-modified IL-2 mutants with different non-natural amino acid (lysine analog) mutations to IL-2 receptor

Referring to Example 3, the phosphorylation of STAT5 was used to evaluate the ability of the PEG-modified IL-2 mutants having a lysine analog mutation prepared in Example 17 to induce signal transduction via the IL-2 receptor.

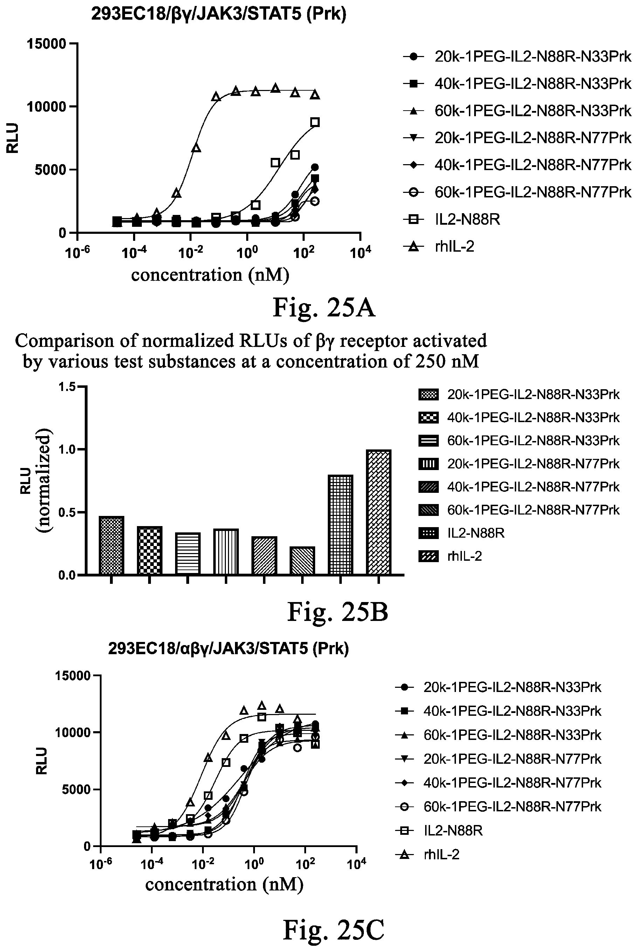

The results are shown in Figure 25 and Table 22. The analysis results show that the PEGylated IL-2 mutant with non-natural amino acid (lysine analog) mutation obtained in Example 17 retained good binding affinity to the IL-2Rαβγ triple receptor, while exhibiting reduced affinity for the IL-2Rβγ dual receptor. Notably, when the PEG molecular weight exceeded 40 k, the STAT5 reporter gene signal via IL-2Rβγ dual receptor at high concentrations was reduced to 15%-30% of that induced by IL-2. This suggests an improved preference for Treg cells, that is, more preference for the activation of Treg cells *in vivo,* while avoiding the activation of effector T cells (Teffs) to a greater extent.

**Table 22 EC50 values for the activation of the IL-2αβγ triple receptor by PEG-IL-2 mutants as determined by a reporter gene assay.**

| Test substance | Activation of IL-2αβγ receptor EC50 (nM) |
|---|---|
| 20k-1PEG-IL2-N88R-N33Prk | 0.3227 |
| 40k-1PEG-IL2-N88R-N33Prk | 0.4426 |
| 60k-1PEG-IL2-N88R-N33Prk | 0.3681 |
| 20k-1PEG-IL2-N88R-N77Prk | 0.3607 |
| 40k-1PEG-IL2-N88R-N77Prk | 0.517 |
| 60k-1PEG-IL2-N88R-N77Prk | 0.4166 |
| IL2-N88R | 0.03056 |
| rh IL-2 | 0.008483 |

## Claims

1. An IL-2 mutant having at least 95% sequence identity with the parent sequence as set forth in SEQ ID NO: 1, and comprising a substitution at one or more positions selected from: N33, S75, N77, D84, N88, V91 and D109 relative to the sequence as set forth in SEQ ID NO: 1.

2. The IL-2 mutant according to claim 1, wherein the position is substituted with a natural amino acid or a non-natural amino acid.

3. The IL-2 mutant of any one of claims 1 to 2, wherein the non-natural amino acid is selected from: a lysine analog, a cysteine analog or a histidine analog, a phenylalanine analog, a non-natural amino acid comprising an aromatic side chain, a non-natural amino acid comprising an azide group, a non-natural amino acid comprising an alkyne group, or a non-natural amino acid comprising an aldehyde or ketone group.

4. The IL-2 mutant according to any one of claims 1 to 3 , wherein the non-natural amino acid is selected from a phenylalanine analog or a lysine analog, preferably, the phenylalanine analog is p-acetylphenylalanine, and the lysine analog is N-E-propargyloxycarbonyl-L-lysine.

5. The IL-2 mutant of any one of claims 1 to 4 , wherein the substitution is one or more selected from N33C, S75C, N77C, D84C, N88R, N88C, V91C and D109C; or one or more selected from N33 pAcF, N77 pAcF or N88R; or one or more selected from N33Prk, N77Prk or N88R.

6. The IL-2 mutant of any one of claims 1 to 5 , wherein the substitution comprises a combination of a substitution of N88 and a substitution selected from any one of N33, S75, N77, D84, V91 and D109.

7. The IL-2 mutant of any one of claims 1 to 6 , wherein the substitution comprises a combination of a substitution of N88R and a substitution selected from any one of N33, S75, N77, D84, V91 and D109, wherein the amino acid substitution other than N88R is modified with a water-soluble polymer, for example, the water-soluble polymer is a PEG molecule.

8. The IL-2 mutant according to claim 7, wherein the PEG molecule can be a linear molecule or a branched molecule.

9. The IL-2 mutant according to claim 7 or 8, wherein the PEG molecule has an average molecular weight of 5-100K Da, preferably 20K~100K, more preferably 40k~80k.

10. The IL-2 mutant of any one of claims 7 to 9, wherein the PEG molecule comprises a chemically reactive group for conjugation with a protein, such as a maleimide group, a hydroxylamine group, an azide group.

11. The IL-2 mutant of any one of claims 1-10, wherein the IL-2 mutant comprises a substitution of N88R and a PEG molecule is conjugated to the substituted residue at position 77.

12. The IL-2 mutant of claim 11, wherein the substituted residue at position 77 is selected from cysteine, p-acetylphenylalanine or N-E-propargyloxycarbonyl-L-lysine.

13. A pharmaceutical composition comprising the IL-2 mutant according to any one of claims 1 to 12.

14. A nucleic acid molecule encoding the IL-2 mutant according to any one of claims 1 to 12.

15. A vector comprising the nucleic acid molecule according to claim 14.

16. A host cell comprising the nucleic acid molecule of claim 14 or the vector of claim 15.

17. A method for preparing the IL-2 mutant according to any one of claims 1 to 12, including:
1) cultivating the host cell of claim 16 under conditions suitable for expressing the IL-2 mutant;
2) recovering the expressed mutants; and
3) modifying the IL-2 mutant with a water-soluble polymer at a specific site, for example, PEGylation at a specific site.

18. A method for treating a disease or reducing organ transplant rejection in a subject, including administering an effective amount of the IL-2 mutant according to any one of claims 1 to 12 or the pharmaceutical composition according to claim 13 to the subject.

19. Use of the IL-2 mutant according to any one of claims 1 to 12 or the pharmaceutical composition according to claim 13 in the preparation of a medicament for treating a disease or reducing organ transplant rejection.

20. The method of claim 18 or the use of claim 19, wherein the disease is an inflammatory disease (e.g., autoimmune disease, ankylosing spondylitis, amyotrophic lateral sclerosis, hepatitis C associated vasculitis, sclerosing cholangitis, inflammatory myopathy, relapsing polychondritis, Behcet's disease), coronary artery disease, macrophage activation syndrome, acute lung injury, hypertension, Takayasu disease, Duchenne muscular dystrophy, transient ischemic stroke, ischemic heart disease, Wiskott-Aldrich Syndrome, bone marrow transplantation, or transplantation indications (such as corneal transplantation, islet transplantation, skin transplantation), preferably, the autoimmune disease is rheumatoid arthritis, autoimmune encephalitis, type I diabetes, nephritis, multiple sclerosis, systemic lupus erythematosus, cutaneous lupus erythematosus, lupus nephritis, Sjögren's syndrome, psoriasis, plaque psoriasis, alopecia areata, dermatomyositis, scleroderma, myasthenia gravis, demyelinating disease, inflammatory bowel disease, ulcerative colitis, Crohn's disease, autoimmune glomerulonephritis, pulmonary renal hemorrhagic syndrome, graft-versus-host disease, organ transplant rejection, autoimmune hepatitis, autoimmune vasculitis, atopic dermatitis, autoimmune hemolytic anemia, idiopathic thrombocytopenic purpura or idiopathic leukopenia.

21. A method for improving IL-2 receptor activation preference and extending the half-life of an IL-2 mutant, including:
1) substituting asparagine at position 77 of IL-2 with a natural amino acid or an unnatural amino acid; and
2) site-specifically modifying the substituted amino acid at position 77 with a water-soluble polymer, thereby obtaining an IL-2 mutant;
3) optionally, recovering the IL-2 mutant.

22. The method according to claim 21, wherein the natural amino acid or unnatural amino acid is selected from cysteine, p-acetylphenylalanine or N-E-propargyloxycarbonyl-L-lysine.

23. The method of claims 21 to 22, wherein the water-soluble polymer is PEG.

24. The method of claim 23 , wherein the PEG molecule can be a linear molecule or a branched molecule.

25. The method of claim 23 or 24, wherein the PEG molecule has an average molecular weight of 5-100K Da, preferably 20K~100K, more preferably 40k~80k.

26. The method of any one of claims 23 to 25, wherein the PEG molecule comprises a chemically reactive group for conjugation to a protein, such as a maleimide group, a hydroxylamine group, or an azide group.
